# EUROPEAN PATENT SPECIFICATION

(11) **EP 1 773 315 B1**
(45) Date of publication and mention of the grant of the patent: **16.01.2008**
(21) Application number: 05758960.8
(22) Date of filing: 19.07.2005
(51) Int. Cl.: A61K 31/215, A61P 25/28

(54) **ARYLACETIC ACIDS AND RELATED COMPOUNDS FOR TREATMENT OF ALZHEIMER'S DISEASE**
ARYLESSIGSÄUREN UND VERWANDTE VERBINDUNGEN ZUR BEHANDLUNG VON ALZHEIMER-KRANKHEIT
ACIDES ARYLACETIQUES ET COMPOSES ASSOCIES UTILES POUR LE TRAITEMENT DE LA MALADIE D'ALZHEIMER

(30) Priority: 23.07.2004 GB 0416508
(43) Date of publication of application: 18.04.2007
(73) Proprietor: Merck Sharp & Dohme Limited, Hoddesdon, Hertfordshire EN11 9BU (GB)
(72) Inventor: BLURTON, Peter, Harlow Essex CM20 2QR (GB); BURKAMP, Frank, Harlow Essex CM20 2QR (GB); CHURCHER, Ian, Harlow Essex CM20 2QR (GB); HARRISON, Timothy, Harlow Essex CM20 2QR (GB); NEDUVELIL, Joseph, Harlow Essex CM20 2QR (GB)
(74) Representative: Buchan, Gavin MacNicol
(86) International application number: PCT/GB2005/050114
(87) International publication number: WO 2006/008558

(56) References cited:
- WO-A-99/11605
- WO-A-20/04039753
- FR-A- 2 838 439
- DATABASE WPI Section Ch, Week 199708 Derwent Publications Ltd., London, GB; Class B05, AN 1997-083408 XP002343714 TOMOKO ET AL: "Substituted terphenyl derivative and antithrombotic agent containing the same" & JP 08 325182 A (FUJI PHARM IND CO LTD) 10 December 1996 (1996-12-10)

## Description

This invention relates to compounds for use in therapeutic treatment of the human body. In particular, it provides arylacetic acids and related compounds useful for treating diseases associated with the deposition of β-amyloid peptide in the brain, such as Alzheimer's disease, or of preventing or delaying the onset of dementia associated with such diseases.

Alzheimer's disease (AD) is the most prevalent form of dementia. Its diagnosis is described in the Diagnostic and Statistical Manual of Mental Disorders, 4th ed., published by the American Psychiatric Association (DSM-IV). It is a neurodegenerative disorder, clinically characterized by progressive loss of memory and general cognitive function, and pathologically characterized by the deposition of extracellular proteinaceous plaques in the cortical and associative brain regions of sufferers. These plaques mainly comprise fibrillar aggregates of β-amyloid peptide (Aβ). Aβ is formed from amyloid precursor protein (APP) via separate intracellular proteolytic events involving the enzymes β-secretase and γ-secretase. Variability in the site of the proteolysis mediated by γ-secretase results in Aβ of varying chain length, e.g. Aβ(1-38), Aβ(1-40) and Aβ(1-42). N-terminal truncations such as Aβ(4-42) are also found in the brain, possibly as a result of variability in the site of proteolysis mediated by β-secretase. For the sake of convenience, expressions such as "'Aβ( 1-40)" and "Aβ(1-42)" as used herein are inclusive of such N-terminal truncated variants. After secretion into the extracellular medium, Aβ forms initially-soluble aggregates which are widely believed to be the key neurotoxic agents in AD (see Gong et al, PNAS, 100 (2003), 10417-22), and which ultimately result in the insoluble deposits and dense neuritic plaques which are the pathological characteristics of AD.

Other dementing conditions associated with deposition of Aβ in the brain include cerebral amyloid angiopathy, hereditary cerebral haemorrhage with amyloidosis, Dutch-type (HCHWA-D), multi-infarct dementia, dementia pugilistica and Down syndrome.

Various interventions in the plaque-forming process have been proposed as therapeutic treatments for AD (see, for example, Hardy and Selkoe, Science, 297 (2002), 353-6). One such method of treatment that has been proposed is that of blocking or attenuating the production of Aβ for example by inhibition of β- or γ-secretase. It has also been reported that inhibition of glycogen synthase kinase-3 (GSK-3), in particular inhibition of GSK-3α, can block the production of Aβ (see Phiel et al, Nature, 423 (2003), 435-9).

Other proposed methods of treatment include administering a compound which blocks the aggregation of Aβ, and administering an antibody which selectively binds to Aβ.

Another proposed treatment is that of modulation of the action of γ-secretase so as to selectively attenuate the production of Aβ(1-42). This results in preferential secretion of the shorter chain isoforms of Aβ, which are believed to have a reduced propensity for self-aggregation and plaque formation, and hence are more easily cleared from the brain, and/or are less neurotoxic. Compounds showing this effect include certain non-steroidal antiinflammatory drugs (NSAIDs) and their analogues (see WO 01/78721 and US 2002/0128319 and Weggen et al Nature, 414 (2001) 212-16; Morihara et al, J. Neurochem., 83 (2002), 1009-12; and Takahashi et al, J. Biol. Chem., 278 (2003), 18644-70). Compounds which modulate the activity of PPARα and/or PPARδ are also reported to have the effect of lowering Aβ(1-42) (WO 02/100836). NSAID derivatives capable of releasing nitric oxide have been reported to show improved anti-neuroinflammatory effects and/or to reduce intracerebral Aβ deposition in animal models (WO 02/092072; Jantzen et al, J. Neuroscience, 22 (2002), 226-54). US 2002/0015941 teaches that agents which potentiate capacitative calcium entry activity can lower Aβ(1-42).

Japanese Patent Application No. 08-325182 discloses certain tetphenyl-substituted alkanoic acid derivatives (including acetic acid derivatives) as antithrombotic agents. There is no disclosure or suggestion of any effect on the secretion of Aβ, or of any utility in the treatment or prevention of AD or any other disorders associated with deposition of Aβ in the brain.

It has now been found that certain substituted arylacetic acids and related compounds have the desirable property of selectively inhibiting production of Aβ (1-42).

According to the present invention there is provided the use, for the manufacture of a medicament for treatment or prevention of a disease associated with the deposition of β-amyloid in the brain, of a compound of formula I: 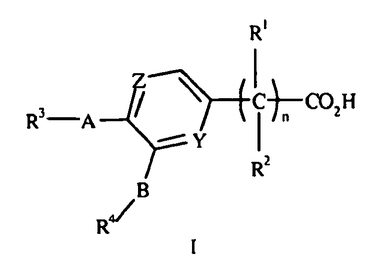 or a pharmaceutically acceptable salt thereof;
wherein n is 0, 1 or 2;
Y is N or CH;
Z is N or CR⁶;
A and B independently represent a bond or a divalent linking group comprising a chain of 1-3 atoms selected from C, O, N and S, with the proviso that not more than one of said atoms is O, N or S;
R¹ and R² independently represent H, F, OR⁵ or R⁵, or together complete a C₃₋₆cycloalkyl group which optionally bears a C₁₋₄alkyl substituent;
R³ and R⁴ independently represent acyclic hydrocarbon groups of 5-10 carbon atoms or mono-or bi-cyclic ring systems comprising 5 to 10 ring atoms selected from C, N, O and S, provided that not more than 3 ring atoms in any single ring are other than C, said ring system optionally bearing up to 3 substituents selected from halogen, N₃, CN, NO₂, R⁵, OR⁵, SR⁵, CO₂R⁵, OCOR⁵ and COR⁵;
R⁵ represents a hydrocarbon group of up to 7 carbon atoms which is optionally substituted with up to 3 halogen atoms; and
R⁶ represents H, or has the same definition as R³.

In a particular embodiment, A and B independently represent a bond or a divalent linking group comprising a chain of 1-3 atoms selected from C, O and S, with the proviso that not more than one of said atoms is O or S, and
R¹ and R² independently represent H, F, or R⁵, or together complete a C₃₋₆cycloalkyl group; and
R³ and R⁴ independently represent hydrocarbon groups of 6-10 carbon atoms or mono-or bi-cyclic ring systems comprising 5 to 10 ring atoms selected from C, N, O and S, provided that not more than 3 ring atoms in any single ring are other than C, said ring system optionally bearing up to 3 substituents selected from halogen, CN, NO₂, R⁵, OR⁵, SR⁵, CO₂R⁵, OCOR⁵ and COR⁵.

Where a variable occurs more than once in formula I, the identity taken by said variable at any particular occurrence is independent of the identity taken at any other occurrence.

The disease associated with deposition of Aβ in the brain is typically Alzheimer's disease (AD), cerebral amyloid angiopathy, multi-infarct dementia, dementia pugilistica or Down syndrome, preferably AD.

In a second aspect, the invention provides the use of a compound of Formula I as defined above, or a pharmaceutically acceptable salt thereof, in the manufacture of a medicament for treating, preventing or delaying the onset of dementia associated with Alzheimer's disease, cerebral amyloid angiopathy, HCHWA-D, multi-infarct dementia, dementia pugilistica or Down syndrome.

The invention also provides a method of treating or preventing a disease associated with deposition of Aβ in the brain comprising administering to a patient in need thereof a therapeutically effective amount of a compound of Formula I as defined above or a pharmaceutically acceptable salt thereof.

In a further aspect, the invention provides a method of treating, preventing or delaying the onset of dementia associated with Alzheimer's disease, cerebral amyloid angiopathy, HCHWA-D, multi-infarct dementia, dementia pugilistica or Down syndrome comprising administering to a patient in need thereof a therapeutically effective amount of a compound of Formula I as defined above or a pharmaceutically acceptable salt thereof.

The compounds of Formula I modulate the action of γ-secretase so as to selectively attenuate production of the (1-42) isoform of Aβ without signifcantly lowering production of the shorter chain isoforms such as Aβ(1-40). This results in secretion of Aβ which has less tendency to self-aggregate and form insoluble deposits, is more easily cleared from the brain, and/or is less neurotoxic. Therefore, a further aspect of the invention provides a method for retarding, arresting or preventing the accumulation of Aβ in the brain comprising administering to a subject in need thereof a therapeutically effective amount of a compound of Formula I as defined above or a pharmaceutically acceptable salt thereof.

Because the compounds of formula I modulate the activity of γ-secretase, as opposed to suppressing said activity, it is believed that the therapeutic benefits described above will be obtained with a reduced risk of side effects, e.g. those that might arise from a disruption of other signalling pathways (e.g. Notch) which are controlled by γ-secretase.

In one embodiment of the invention, the compound of Formula I is administered to a patient suffering from AD, cerebral amyloid angiopathy, HCHWA-D, multi-infarct dementia, dementia pugilistica or Down syndrome, preferably AD.

In an alternative embodiment of the invention, the compound of Formula I is administered to a patient suffering from mild cognitive impairment or age-related cognitive decline. A favourable outcome of such treatment is prevention or delay of the onset of AD. Age-related cognitive decline and mild cognitive impairment (MCI) are conditions in which a memory deficit is present, but other diagnostic criteria for dementia are absent (Santacruz and Swagerty, American Family Physician, 63 (2001), 703-13). (See also ''The ICD-10 Classification of Mental and Behavioural Disorders", Geneva: World Health Organisation, 1992, 64-5). As used herein, "age-related cognitive decline" implies a decline of at least six months' duration in at least one of: memory and learning; attention and concentration; thinking; language; and visuospatial functioning and a score of more than one standard deviation below the norm on standardized neuropsychologic testing such as the MMSE. In particular, there may be a progressive decline in memory. In the more severe condition MCI, the degree of memory impairment is outside the range considered normal for the age of the patient but AD is not present The differential diagnosis of MCI and mild AD is described by Petersen et al., Arch. Neurol., 56 (1999), 303-8. Further information on the differential diagnosis of MCI is provided by Knopman et al, Mayo Clinic Proceedings, 78 (2003), 1290-1308. In a study of elderly subjects, Tuokko et al (Arch, Neurol., 60 (2003) 577-82) found that those exhibiting MCI at the outset had a three-fold increased risk of developing dementia within 5 years.

Grundman et al (J. Mol. Neurovci., 19 (2002), 23-28) report that lower baseline hippocampal volume in MCI patients is a prognostic indicator for subsequent AD. Similarly, Andreasen et al (Acta Neurol. Scand, 107 (2003) 47-51) report that high CSF levels of total tau, high CSF levels of phospho-tau and lowered CSF levels of Aβ42 are all associated with increased risk of progression from MCI to AD.

Within this embodiment, the compound of Formula I is advantageously administered to patients who suffer impaired memory function but do not exhibit symptoms of dementia. Such impairment of memory function typically is not attributable to systemic or cerebral disease, such as stroke or metabolic disorders caused by pituitary dysfunction. Such patients may be in particular people aged 55 or over, especially people aged 60 or over, and preferably people aged 65 or over. Such patients may have normal patterns and levels of growth hormone secretion for their age. However, such patients may possess one or more additional risk factors for developing Alzheimer's disease. Such factors include a family history of the disease; a genetic predisposition to the disease; elevated serum cholesterol; and adult-onset diabetes mellitus.

In a particular embodiment of the invention, the compound of Formula I is administered to a patient suffering from age-related cognitive decline or MCI who additionally possesses one or more risk factors for developing AD selected from: a family history of the disease; a genetic predisposition to the disease; elevated serum cholesterol; adult-onset diabetes mellitus; elevated baseline hippocampal volume; elevated CSF levels of total tau; elevated CSF levels of phospho-tau; and lowered CSF levels of Aβ(1-42),

A genetic predisposition (especially towards early onset AD) can arise from point mutations in one or more of a number of genes, including the APP, presenilin-1 and presenilin-2 genes. Also, subjects who are homozygous for the e4 isoform of the apolipoprotein E gene are at greater risk of developing AD.

The patient's degree of cognitive decline or impairment is advantageously assessed at regular intervals before, during and/or after a course of treatment in accordance with the invention, so that changes therein may be detected, e.g. the slowing or halting of cognitive decline. A variety of neuropsychological tests are known in the art for this purpose, such as the Mini-Mental State Examination (MMSE) with norms adjusted for age and education (Folstein et al., J. Psych. Res., 12 (1975), 196-198, Anthony et al., Psychological Med., 12 (1982), 397-408; Cockrell et al., Psychopharmacology, 24 (1988), 689-692; Crum et al., J. Am. Med. Assoc'n. 18 (1993), 2386-2391). The MMSE is a brief, quantitative measure of cognitive status in adults. It can be used to screen for cognitive decline or impairment, to estimate the severity of cognitive decline or impairment at a given point in time, to follow the course of cognitive changes in an individual over time, and to document an individual's response to treatment. Another suitable test is the Alzheimer Disease Assessment Scale (ADAS), in particular the cognitive element thereof (ADAS-cog) (See Rosen et al., Am. J. Psychiatry, 141 (1984), 1356-64).

According to a further aspect of the invention, there is provided a compound according to formula I as defined above, or a pharmaceutically acceptable salt thereof, with the proviso that when n is I or 2, Y and Z are CH, and A and B both represent a bond, and at least one of R¹ and R² is H, then R³ and R⁴ do not both represent unsubstituted phenyl.

As used herein, the expression "hydrocarbon group" refers to groups consisting solely of carbon and hydrogen atoms. Such groups may comprise linear, branched or cyclic structures, singly or in any combination consistent with the indicated maximum number of carbon atoms, and may be saturated or unsaturated, including aromatic when the indicated maximum number of carbon atoms so permits, unless otherwise indicated.

As used herein, the expression ''C₁₋ₓalkyl'' where x is an integer greater than 1 refers to siraight-chained and branched alkyl groups wherein the number of constituent carbon atoms is in the range 1 to x. Particular alkyl groups are methyl, ethyl, n-propyl, isopropyl and t-butyl. Derived expressions such as ''C₂₋₆alkenyl'', "hydroxyC₁₋₆alkyl", "heteroarylC₁₋₆alkyl", "C₂₋₆alkynyl" and "C₁₋₆alkoxy" are to be construed in an analogous manner.

The expression "C₃₋₆cycloalkyl" refers to cyclic non-aromatic hydrocarbon groups containing from 3 to 6 ring carbon atoms. Examples include cyclopropyl, cyclobutyl, cyclopentyl and cyclohexyl.

The term "halogen" as used herein includes fluorine, chlorine, bromine and iodine, of which fluorine and chlorine are preferred unless otherwise indicated.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include salts formed by neutralisation of the carboxylic acid group with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

In formula I, n is preferably 1 or 2, and most preferably n is 1.

Y represents CH or N, and Z represents CR⁶ or N. Preferably Y and Z are not both N. Typical identities for R⁶ include H and optionally substituted phenyl, such as 4-trifluoromethyl phenyl. In a preferred embodiment Y and Z are both CH.

A and B independently represent a bond or a divalent linking group comprising a chain or 1-3 atoms selected from C, N, O and S, provided that not more than one of said chain atoms is N, O or S. Preferred linking groups comprise one or two chain atoms. Examples of suitable linking groups include CH₂, O, S, CH₂CH₂, CH=CH, C≡C, NH, N(R⁵), C(=O), C(=CHR⁵), OCH₂, CH₂O, SCH₂ CH₂S and CH₂CH₂CH₂, where R⁵ has the same meaning as before. In this context, R⁵ preferably represents C ₁₋₆alkyl or C₂₋₆alkenyl.

Examples of preferred linking groups include O, OCH₂, C≡C, NH, N(CH₂CH=CMe₂, C(=O) and C(=CHCH₂CHMe₂). (For the avoidance of doubt, linking groups are depicted with the attachment point of R³ or R⁴ on the right).

In a preferred embodiment, one of A and B (preferably B) represents a bond and the other represents a bond or a linking group as defined above. In a further preferred embodiment, A and B each represents a bond.

R¹and R² independently represent H, F, R⁵ or OR⁵ (where R⁵ is as defined previously), or together complete a C₃₋₆cycloalkyl group which optionally bears a C₁₋₄alkyl substituent (such as methyl). Suitable identities for R⁵ in this context include C₁₋₆alkyl (such as methyl, ethyl, n-propyl, isopropyl, n-butyl, isobutyl, t-butyl, n-pentyl, isopentyl, n-hexyl and 4-methylpentyl), halo-C₁₋₆alkyl (such as 3,3,3-trifluoropropyl, 4-chlorobutyl, 5-chloropentyl and 3-chloropropyl), C₂₋₆ alkenyl (such as allyl and 2-methyl(propen-3-yl), cycloalkylalkyl (such as cyclopropylmethyl) and arylalkyl (such as benzyl). When R¹ and R² together complete a cycloalkyl group, suitable examples include cyclopropyl, cyclobutyl, methylcyclobutyl, cyclopentyl and cyclohexyl.

When n is 2, preferably at least one of the CR¹R² groups is CH₂.

In a preferred embodiment, n is 1 and either one of R¹ and R² is H and the other is H, R⁵ or OR⁵, or R¹ and R² together complete a cycloalkyl group. More preferably, n is 1, R¹ is H and R² is C₁₋₆alkyl, halo-C₁₋₆alkyl or C₂₋₆alkenyl.

In one embodiment, one or both of R³ and R⁴ represents an acyclic hydrocarbon group of 5-10 carbon atoms, such as a branched alkyl group (e.g. 4-methylpentyl or 3-methylbutyl) or alkenyl group (e.g. 3-methylbut-2-enyl). In such cases, A and/or B (as appropriate) suitably represents a bond, O, NH or N(R⁵).

In another embodiment one or both of R³ and R⁴ represents a mono- or bicyclic ring systems as defined previously. Said ring system may be saturated or unsaturated, including aromatic and heteroaromatic. Examples of suitable monocyclic systems include phenyl, pyridyl, piperazinyl, piperidinyl, morpholinyl, cyclopentyl, cyclohexyl, cyclohexenyl and cycloheptyl. Examples of suitable bicyclic systems include naphthyl, quinolinyl, isoquinolinyl, indenyl and the partially- or fully-hydrogenated derivatives thereof. Preferably at least one ring system represented by R³ and R⁴ (in particular, R⁴) is aromatic or heteroaromatic, especially phenyl. In a particular embodiment, R³ and R⁴ both represent optionally-substituted phenyl.

Ring systems represented by R³ and/or R⁴ preferably bear at least one, and preferably not more than two, substituents as defined previously. Preferred substituents include halogen, N₃, R⁵ and OR⁵ where R⁵ is as defined previously. Typical identities for R⁵ in this context include C₁₋₆alkyl which is optionally substituted with up to 3 halogen atoms (such as methyl, ethyl, n-propyl, isopropyl, n-butyl, t-butyl and CF₃) and phenyl.

In a further aspect, the invention provides a compound of formula II: 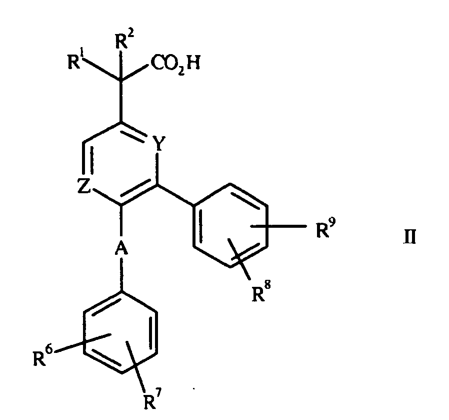 or a pharmaceutically acceptable salt thereof;
wherein R⁶, R⁷, R⁸ and R⁹ are independently selected from H, halogen, R⁵ and OR⁵ provided at least one of R⁶-R⁹ is other than H;
and A, Y, Z, R¹, R² and R⁵ have the same definitions and preferred identities as before.

In one preferred subset of the compounds of formula II, R⁶ represents CF₃ and is preferably in the 4-position and R⁷ represents H, halogen, N₃, C₁₋₄alkyl or CF₃.

In another preferred subset of the compounds of formula II, R⁸ represents CF₃ and is preferably in the 4-position and R⁹ represents H, halogen or CF₃.

Specific examples of compounds in accordance with formula II include those in which Y and Z are each CH, and A, R¹, R², R⁶-R⁹ are as indicated in the following table:

| **A** | **R⁶/R⁷** | **R⁸/R⁹** | **R¹** | **R²** |
|---|---|---|---|---|
| bond | 4-CF₃ | 4-isopropyl | H | H |
| bond | 2,4-di-CF₃ | 4-isopropyl | H | H |
| bond | 4-isopropyl | 4-isopropyl | H | H |
| bond | 4-t-butyl | 4-isopropyl | H | H |
| bond | 4-CF₃ | 4-t-butyl | H | H |
| bond | 4-CF₃ | 4-n-butyl | H | H |
| bond | 3-methyl | 4-isopropyl | H | H |
| bond | 4-methyl | 4-isopropyl | H | H |
| bond | 4-(2-methylpropyl) | 4-isopropyl | H | H |
| bond | 2,4-di-CF₃ | 4-n-butyl | H | H |
| bond | 3-CF₃ | 4-isopropyl | H | H |
| bond | 4-PhO | 4-isopropyl | H | H |
| bond | 4-n-butyl | 4-isopropyl | H | H |
| bond | 2,4-di-Cl | 4-isopropyl | H | H |
| bond | 4-isopropoxy | 4-isopropyl | H | H |
| bond | 4-CF₃ | 4-CF₃ | H | H |
| O | 4-CF₃ | 4-isopropyl | H | H |
| OCH₂ | 4-CF₃ | 4-isopropyl | H | H |
| C≡C | H | 4-isopropyl | H | H |
| C≡C | 4-CF₃ | 4-isopropyl | H | H |
| bond | 4-CF₃ | 4-CF₃ | methyl | methyl |
| bond | 4-CF₃ | 4-CF₃ | allyl | allyl |
| bond | 4-CF₃ | 4-CF₃ | cyclopentyl | |
| bond | 4-CF₃ | 4-CF₃ | H | methyl |
| bond | 4-CF₃ | 4-CF₃ | H | n-butyl |
| bond | 4-CF₃ | 4-CF₃ | H | benzyl |

Further specific examples of compounds in accordance with formula II include those in which Y and Z are each CH, and A, R¹, R² and R⁶-R⁹ are as indicated in the following table:

| **A** | **R⁶/R⁷** | **R⁸/R⁹** | **R¹** | **R²** |
|---|---|---|---|---|
| bond | 4-CF₃ | 4-CF₃ | H | ethyl |
| bond | 4-CF₃ | 4-CF₃ | H | n-propyl |
| bond | 4-CF₃ | 4-CF₃ | H | 4-Me-pentyl |
| bond | 4-CF₃ | 4-CF₃ | H | n-butyl |
| bond | 4-CF₃ | 4-CF₃ | cyclopropyl | |
| bond | 4-CF₃ | 4-CF₃ | cyclohexyl | |
| bond | 4-CF₃ | 4-CF₃ | cyclobutyl | |
| bond | 4-CF₃ | 2,4-di-CF₃ | H | n-propyl |
| bond | 4-CF₃ | 4-CF₃ | H | isopropyl |
| bond | 4-CF₃ | 4-CF₃ | H | cyclopropylmethyl |
| bond | 4-CF₃ | 4-CF₃ | H | 3,3,3-trifluoroethyl |
| bond | 4-CF₃ | 4-CF₃ | H | 4-chlorobutyl |
| bond | 4-CF₃ | 4-CF₃ | H | 2-Me-propen-3-yl |
| bond | 4-CF₃ | 2,4-di-CF₃ | H | isobutyl |
| bond | 4-CF₃ | 2,4-di-CF₃ | cyclopentyl | |
| bond | 2,4-di-CF₃ | 4-CF₃ | H | isobutyl |
| bond | 4-CF₃ | 4-CF₃ | H | 3-chloropropyl |
| bond | 4-CF₃ | 4-CF₃ | H | 5-chloropentyl |
| bond | 4-CF₃ | 4-CF₃ | H | isopropoxy |
| NH | 4-t-butyl | 4-CF₃ | H | isobutyl |
| bond | 4-CF₃ | 4-CF₃ | 3-Me-cyclobutyl | |
| bond | 2-Cl-4-CF₃ | 4-CF₃ | H | isobutyl |
| bond | 2-Cl-4-CF₃ | 4-CF₃ | H | 2-Me-propen-3-yl |
| bond | 4-CF₃ | 4-CF₃ | H | ethoxy |
| bond | 4-CF₃ | 4-CF₃ | H | isopentyloxy |
| bond | 4-CF₃ | 4-CF₃ | H | isobutyl |
| bond | 4-CF₃ | 2,4-di-CF₃ | H | H |
| bond | 4-CF₃ | 4-OCF₃ | H | H |
| bond | 4-CF₃ | 2-Cl-4- CF₃ | H | H |
| bond | 4-CF₃ | 3,5-di-CF₃ | H | H |
| bond | 2,4-CF₃ | 4-CF₃ | H | H |
| bond | 4-CF₃ | 2-F-4- CF₃ | H | H |
| NH | 4-CF₃ | 4-CF₃ | H | H |
| C(O) | 4-CF₃ | 4-CF₃ | H | H |
| bond | 2-Cl-4-CF₃ | 4-CF₃ | H | H |
| bond | 2-ⁿpropyl-4-CF₃ | 4-CF₃ | H | H |
| C(=CHCH₂CHMe₂) | 4-CF₃ | 4-CF₃ | H | H |
| bond | 4-Br | 4-CF₃ | H | H |
| bond | 2-N₃-4-CF₃ | 4-CF₃ | H | H |
| bond | 2-N₃-4-CF₃ | 4-CF₃ | H | isobutyl |

Further compounds in accordance with the invention include those of the formula: 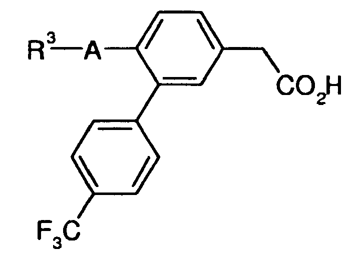 where A and R³ are as shown in the following table:

| **A** | **R³** |
|---|---|
| bond | 6-CF₃-pyridin-3-yl |
| bond | 4-Me-pentyn-1-yl |
| bond | 4-Me-penten-1-yl |
| bond | 4-Me-pentyl |
| N(CH₂CH=CMe₂) | 3-Me-but-2-en-1-yl |
| NH | isopentyl |
| O | 4-Me-pentyl |

The compounds of formula I may be prepared using standard synthetic techniques known to those skilled in the art.
For example, the compounds of formula I are typically prepared by hydrolysis of the corresponding esters I': 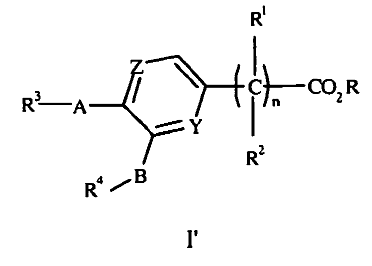 where R represents C₁₋₄alkyl (e.g. methyl) and n, A, B, Y, Z, R¹, R² and R⁴ have the same meanings as before. The hydrolysis may be carried out by heating in aqueous alkali, e.g. in 40% KOH at 100°C.

The compounds of formula Γ in which A represents a bond, CH₂, CH₂CH₂, CH₂CH₂CH₂ or CH=CH may be prepared by reaction of a compound of formula (1a) or (1c) with a boronic acid R³-A'-B(OH)₂: 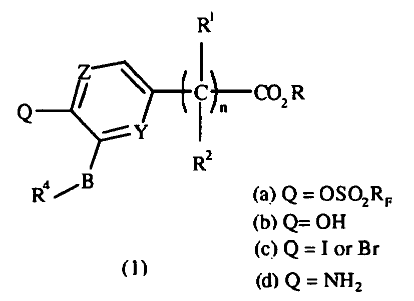 where A' represents a bond, CH₂, CH₂CH₂, CH₂CH₂CH₂ or CH=CH, R_{F} represents perfluoroalkyl of up to 6 carbon atoms (e.g. CF₃) and n, B, Y, Z, R¹, R² and R⁴ have the same meanings as before. The reaction takes place at elevated temperature in a hydrocarbon or ether solvent (eg. toluene or dioxan) in the presence of an inorganic base (e.g. an alkali metal carbonate or phosphate, such as K₃PO₄) and a Pd catalyst (preferably Pd (dppf)Cl₂). A corresponding boronate ester (e.g. a cyclic ester such as the pinacol ester) may be used in place of the boronic acid R³-A'-B(OH)₂.

Compounds (1a) are available from the corresponding phenols (1b) by treatment with (R_{F}SO₂)₂O or R_{F}SO₂Cl in an inert solvent such as dichloromethane at 0°C in the presence of a base such as pyridine.

Compounds I' in which A represents a bond, Z represents CR⁶ and R⁶ has the same identity as R³ may be obtained by bromination of phenols (1b) (Z= CH) to provide the corresponding bromophenols in which Z = CBr, then perfluoroalkylsulphonation and reaction with 2 equivalents of R³-B(OH)₂ as before.

Compounds of formula (1c) are obtainable by diazotisation of anilines (1d) and treatment of the resulting diazonium salts with bromide or iodide ion.

Compounds of formula I' in which A represents O or NH may be obtained, respectively, by reaction of phenols (1b) or anilines (1d) with R³-B(OH)₂. The reaction takes place in the presence of copper (II) acetate, molecular sieves and triethylamine in an inert solvent such as dichloromethane.

Compounds of formula I' in which A represents OCH₂ or NHCH₂ may be obtained, respectively, by reaction of phenols (1b) or anilines (1d) with R³-CH₂-L (where L is a leaving group such as halide, e.g. Br). The reaction takes place in an inert solvent such as DMF in the presence of a base such as potassium carbonate.

Compounds of formula I' in which A comprises N(R⁵) may be prepared from the corresponding compounds in which A comprises NH by alkylation with R⁵-L where L has the same meaning as before, e.g. in an inert solvent such as DMF in the presence of a base such as potassium carbonate.

Compounds of formula I' in which A represents S may be obtained by diazotisation of anilines (1d) and treatment of the resulting diazonium salts with R³SH.

Compounds of formula I' in which A represents -C≡C- may be obtained by reaction of compounds (1a) with R³-C≡CH, typically in the presence of Cul, Ph₃P, (Ph₃P)₄Pd(0) and triethylamine.

Compounds of formula I' in which A represents C(=CHR⁵) may be obtained by reaction of bromides or iodides (1c) with a tributylstannyl derivative R³-C(=CHR⁵)-SnBu₃. The reaction may be carried out in a dipolar aprotic solvent such as DMF with heating in the presence of CuI, Ph₃As and a Pd(0) catalyst such as tris(dibenzylidene)dipalladium(0).

Compounds of formula (1b) or (1d) in which B is a bond may be prepared, respectively, by reaction of compounds of formula (2a) or (2b) with boronic acids R⁴-B(OH)₂: 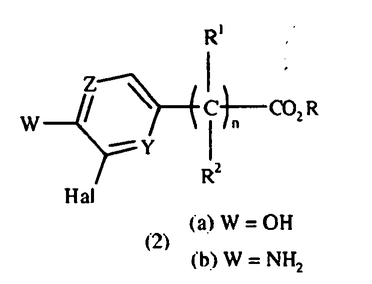 where Hal represents a halogen atom (preferably Br or 1) and n Y, Z, R, R¹, R² and R⁴ have the same meanings as before. The reaction with R⁴-B(OH)₂ takes place under similar conditions to the reaction of compounds (1a) with R³-B(OH)₂, e.g. in dioxan solution at 100°C in the presence of Na₂CO₃ and Pd(dpp)Cl₂, and boronate esters (such as pinacol esters) may be substituted for the boronic acids.

Compounds of formula (1b) and (1d) in which B is other than a bond may be prepared by methods similar to those used for constructing different embodiments of A.

It will be apparent to those skilled in the art that the above-described sequences of steps may be altered by starting from analogues of the compounds (2) in which the attachment positions of W and Hal are reversed, and reversing the order in which R³ and R⁴ are attached.

Compounds of formula I or formula I' in which at least one of R¹ and R² is R⁵ may be obtained from the corresponding compounds of formula I or formula I' in which at least one of R¹ and R² is H by treatment with R⁵-L in the presence of base, where L has the same meaning as before. Use of N-fluorobenzenesulphonimide in place of R⁵-L provides compounds of formula I' in which R¹ and/or R² is F. Similar treatment of compounds of formula I' where R¹ = R² =H with a dihaloalkane such as 1,3-diiodopropane, 1,4-diiodobutane and the like provides compounds of formula r in which R¹ and R² together form a cycloalkyl group.

Compounds of formula I' in which one of R¹ and R² represents OR⁵ may be obtained by treatment of compounds of formula Γ in which R¹ = R² =H with 4-acetamidobenzenesulphonyl azide and reaction of the resulting diazo compound with R⁵OH. The first step may be carried out in acetonitrile at -10°C to ambient, and the second step in refluxing toluene in the presence of a Rh(II) catalyst such as the diacetate dimer dihydrate.

It will also be apparent that individual compounds of formula I prepared by the above routes may be converted into other compounds in accordance with formula 1 by means of standard synthetic techniques. Alternatively, such conversion may be carried out on the synthetic precursors of compounds of formula I.

Where they are not themselves commercially available, the starting materials and reagents employed in the above-described synthetic schemes may be obtained by published routes or the application of standard techniques of organic synthesis to commercially available materials.

Certain compounds according to the invention may exist as optical isomers due to the presence of one or more chiral centres or because of the overall asymmetry of the molecule. Such compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as di-p-toluoyl-D-tartaric acid and/or di-p-toluoyl-L-tartaric acid, followed by fractional crystallisation and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 3rd ed., 1999. The protecting groups may be removed at a convenient subsequent stage using methods known from the art.

The compounds of Formula I are typically used in the form of pharmaceutical compositions comprising one or more compounds of Formula I and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions useful in the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcedulose, methylcellulose, poly(ethylene glycol), poly(vinylpyrrolidone) or gelatin.

For treating or preventing Alzheimer's disease, a suitable dosage level is about 0.01 to 250 mg/kg per day, preferably about 0.01 to 100 mg/kg per day, and more preferably about 0.05 to 50 mg/kg of body weight per day, of the active compound. The compounds may be administered on a regimen of 1 to 4 times per day. In some cases, however, a dosage outside these limits may be used.

The compounds of Formula I optionally may be administered in combination with one or more additional compounds known to be useful in the treatment or prevention of AD or the symptoms thereof. Such additional compounds thus include cognition-enhancing drugs such as acetylcholinesterase inhibitors (e.g. donepezil and galanthamine), NMDA antagonists (e.g. memantine) or PDE4 inhibitors (e.g. Ariflo™ and the classes of compounds disclosed in WO 03/018579, WO 01/46151, WO 02/074726 and WO 02/098878). Such additional compounds also include cholesterol-lowering drugs such as the statins, e.g. simvastatin. Such additional compounds similarly include compounds known to modify the production or processing of Aβ in the brain ("amyloid modifiers"), such as compounds which inhibit the secretion of Aβ (including γ-secrctase inhibitors, β-secretase inhibitors, and GSK-3α inhibitors), compounds which inhibit the aggregation of Aβ, and antibodies which selectively bind to Aβ.

In this embodiment of the invention, the amyloid modifier may be a compound which inhibits the secretion of Aβ, for example an inhibitor of
of γ-secretase (such as those disclosed in WO 01/53255, WO 01/66564, WO 01/70677, WO 01/90084, WO 01/77144, WO 02/30912, WO 02/36555, WO 02/081435, WO 02/081433, WO 03/018543, WO 03/093252, WO 03/093264, WO 03/093251, WO 03/093253, WO 03/013506, WO 03/013527 and WO 03/014075), or a β-secretase inhibitor (such as those disclosed in WO 03/037325, WO 03/030886, WO 03/006013, WO 03/006021, WO 03/006423, WO 03/006453, WO 02/002122, WO 01/70672, WO 02/02505, WO 02/02506, WO 02/02512, WO 02/02520, WO 02/098849 and WO 02/100820), or any other compound which inhibits the formation or release of Aβ Including those disclosed in WO 98/28268, WO 02/47671, WO 99/67221, WO 01/34639, WO 01/34571, WO 00/07995, WO 00/38618, WO 01/92235, WO 01/77086, WO 01/74784, WO 01/74796, WO 01/74783, WO 01/60826, WO 01/19797, WO 01/27108, WO 01/27091, WO 00/50391, WO 02/057252, US 2002/0025955 and US2002/0022621, and also including GSK-3 inhibitors, particularly GSK-3α inhibitors, such as lithium, as disclosed in Phiel et al, Nature, 423 (2003), 435-9.

Within this embodiment, the amyloid modifier is advantageously a γ-secrelase inhibitor, preferred examples of which include a compound of formula XI: 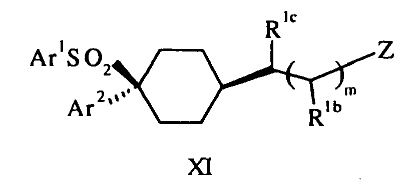 wherein m, Z, R^{1b}, R^{1c}, Ar¹ and Ar² are as defined in WO 03/O18543;
or a pharmaceutically acceptable salt thereof.

Such compounds may be prepared as described in WO 03/018543. Preferred examples include those defined by formula XIa: 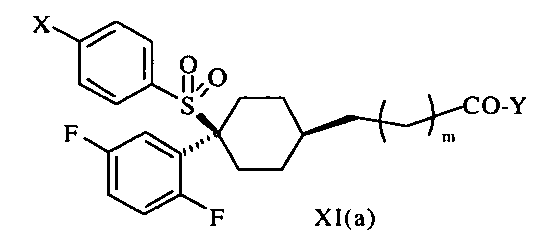 and the pharmaceutically acceptable salts thereof, wherein m is 0 or 1, X is Cl or CF₃, and Y is OH, OC₁₋₆alkyl, NH₂ or NHC ₁₋₆alkyl. Particular examples include those in which m is 1 and Y is OH (or the sodium salts thereof), and those in which m is 0 and Y is NH₂ or NHC₁₋₆alkyl.

Another preferred class of γ-secretase inhibitors for use in this embodiment of the invention is that defined by formula XII: 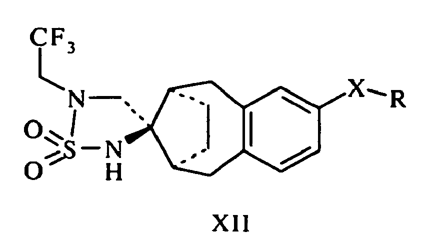 wherein X and R are as defined in WO 03/093252;
or a pharmaceutically acceptable salt thereof.

X is very aptly 5-substituted-thiazol-2-yl, 5-substituted-4-methylthiazol-2-yl, 5-substituted-1-methylpyrazol-3-yl, 1-substituted-imidazol-4-yl or 1-substituted-1,2,4-triazol-3-yl. Preferably, R represents optionally-substituted phenyl or heteroaryl such as phenyl, monohalophenyl, dihalophenyl, trihalophenyl, cyanophenyl, methylphenyl, methoxyphenyl, trifluoromethylphenyl, trifluoromethoxyphenyl, pyridyl, monohalopyridyl and trifluoromethylpyridyl, wherein "halo" refers to fluoro or chloro. Particularly preferred identities of R-X- include 5-(4-fluorophenyl)-1-methylpyrazol-3-yl, 5-(4-chlorophenyl)-1-methylpyrazol-3-yl and 1-(4-fluorophenyl)imidazol-4-yl. Such compounds may be prepared by methods disclosed in WO 03/093252.

Further preferred classes of γ-secretase inhibitors include those disclosed in WO 03/093264, WO 03/093251, WO 03/093253, WO 2004/039370, WO 2004/39800 WO 2004/031139, WO 2005/030731, WO 2005/014553 and WO 2005/101538.

Alternatively, the amyloid modifier may be a compound which inhibits the aggregation of Aβ. Suitable examples include chelating agents such as clioquinol (Gouras and Beal, Neuron, 30 (2001), 641-2) and the compounds disclosed in WO 99/16741, in particular that known as DP-109 (Kalendarev et al, J. Pharm. Biomed. Anal., 24 (2001), 967-75). Other inhibitors of Aβ aggregation suitable for use in the invention include the compounds disclosed in WO 96/28471, WO 98/08868 and WO 00/052048, including the compound known as Apan™ (Praecis); WO 00/064420, WO 03/017994, WO 99/59571 and the compound known as Alzhemed™ (Neurochem); WO 00/149281 and the compositions known as PTI-777 and PTI-00703 (ProteoTech); WO 96/39834, WO 01/83425, WO 01/55093, WO 00/76988, WO 00/76987, WO 00/76969, WO 00/76489, WO 97/26919, WO 97/16194, and WO 97/16191.

Alternatively, the amyloid modifier may be an antibody which binds selectively to Aβ. Said antibody may be polyclonal or monoclonal, but is preferably monoclonal, and is preferably human or humanized. Preferably, the antibody is capable of sequestering soluble Aβ from biological fluids, as described in WO03/016466, WO03/016467, WO 03/015691 and WO 01/62801. Suitable antibodies include humanized antibody 266 (described in WO 01/62801) and the modified version thereof described in WO 03/016466.

As used herein, the expression ''in combination with" requires that therapeutically effective amounts of both the compound of Formula I and the additional compound are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible. When the additional compound is an antibody, it will typically be administered parenterally and separately from the compound of Formula I.

In a further aspect, the invention provides the combination of a compound of formula I or a pharmaceutically acceptable salt thereof and a compound of formula XI(a) or a pharmaceutically acceptable salt thereof for use in treatment or prevention of a disease associated with deposition of β-amyloid in the brain. Said use may involve the simultaneous or separate administration of the respective compounds to a patient in need of such treatment or prevention.

In a further aspect, the invention provides a pharmaceutical composition comprising, in a pharmaceutically acceptable carrier, a compound of formula I or a pharmaceutically acceptable salt thereof and a compound of formula XI(a) or a pharmaceutically acceptable salt thereof. Preferably, the pharmaceutical composition is in a unit dose form suitable for oral administration, such as a tablet or a capsule.

### EXAMPLES

The ability of the compounds of Formula I to selectively inhibit production of Aβ(1-42) was determined using the following assay:

### Cell-based γ-Secretase Assay

Human SH-SY5Y neuroblastoma cells overexpressing the direct γ-secretase substrate SPA4CT were induced with sodium butyrate (10 mM) for 4 hours prior to plating. Cells were plated at 35,000 cells/well/100 µl in 96-well plates in phenol red-free MEM/10% FBS, 50 mM HEPES, 1% Glutamine and incubated for 2 hrs at 37 °C, 5% CO₂.

Compounds for testing were diluted into Me₂SO to give a ten point dose-response curve. Typically 10 µl of these diluted compounds in Me₂SO were further diluted into 182 µl dilution buffer (phenol red-free MEM/10% FBS, 50 mM HEPES, 1% Glutamine) and 10 µl of each dilution was added to the cells in 96-well plates (yielding a final Me₂SO concentration of 0.5%). Appropriate vehicle and inhibitor controls were used to determine the window of the assay.

After incubation overnight at 37 °C, 5%CO₂, 10 µl and 50 µl media were transferred into a fresh Costar round-bottom 96-well plate for detection of Aß(40) and Aß(42) peptides, respectively. 40 µl Origen buffer (PBS, 2% BSA, 0.2% Tween-20) was added to the Aß(40) wells followed by the addition of 25 µl the respective antibody premixes to the wells:
Aβ(40) premix: 1 µg/ml ruthenylated G2-10 antibody, 4 µg/ml
   biotinylated 4G8 antibody diluted in Origen buffer
Aβ(42) premix: 0.5 µg/ml ruthenylated G2-11 antibody, 4 µg/ml
   biotinylated 4G8 antibody diluted in Origen buffer

### (Biotinylated 4G8 antibody supplied by Signet Pathology Ltd; G2-10 and G2-11 antibodies supplied by Chemicon)

After overnight incubation of the assay plates on a shaker at 4 °C, the Origen M8 Analyser (Igen Inc.) was calibrated according to the manufacturer's instructions. 25 µl of streptavidin magnetic bead (Dynal) premix (400 µg/ml streptavidin beads/ml in Origen buffer) was added to the assay plates and incubated on a shaker for 15 minutes. 150 µl Origen buffer was added to each well and the plates were read on the Origen M8 Analyser according to the manufacturer's instructions.

Cell viability was measured in the corresponding cells after removal of the media for the Aβ assays by a colorimetric cell proliferation assay (CellTiter 96™ AQ assay, Promega) utilizing the bioreduction of MTS (Owen's reagent) to formazan according to the manufacturer's instructions. Briefly, 5 µl of lOx MTS/PES was added to the remaining 50 µl of media before returning to the incubator. The optical density was read at 495 nm after ~4 hours.

LD₅₀ and IC₅₀ values for inhibition of Aß(40) and Aß(42) were calculated by nonlinear regression fit analysis using the appropriate software (eg. Excel fit). The total signal and the background were defined by the corresponding Me₂SO and inhibitor controls.
The compounds listed in the Tables above all gave IC₅₀ values for Aβ(1-42) inhibition that were at least 2- fold lower than the corresponding IC₅₀ values for Aβ(1-40) inhibition, typically at least 5-fold lower, and in the preferred cases at least 50-fold lower.

### Exanmle 1

### 3-(4-Isopropylphenyl)-4-(4-(trifluoromethyl)phenyl)phenylacetic acid

### Step 1: Methyl (4-hydroxy-3-(4-isopronylphenyl)phenyl acetate

Methyl (3-bromo-4-hydroxyphenyl)acetate (8.24g, 33.6mmol) (prepared according to WO 9749710) in dioxan (80ml) was treated with 4-isopropylphenyl boronic acid (8.2g, 50mmol) and saturated sodium carbonate solution (45ml). After degassing with nitrogen, [1,1'-diphenylphosphino)ferrocene]dichloropalladium(II), complex with dichloromethane (1.39g, 1.7mmol) was added and the mixture degassed. The reaction was heated for 18 hours at 100°C, cooled to room temperature and water added. The mixture was neutralized using 2N hydrochloric acid and extracted using ethyl acetate (5x100ml). The combined organics were dried over sodium sulphate, filtered and evaporated. Purification by flash chromatography on silica using 5-10% ethyl acetate in isohexane yielded the title compound (6.3g, 66%). ¹H NMR (400MHz, CDCl₃) δ 7.39-7.33 (4H, m), 7.15 (2H, m), 6.93 (1H, dd, *J =* 1.6, 8.8Hz), 5.24 (1H, s), 3.69 (3H, s), 3.58 (2H, s), 2.99-2.91 (1H, m), 1.29 (6H, d, J = 7.0Hz).

### Step 2: Methyl (4'-isopropyl-6-{[(trifluoromethyl)sulfonyl]oxy}biphenyl-3-yl)acetate

Methyl (4-hydroxy-3-(4isopropylphenyl)phenyl acetate (Step 1, 1.42g), 5mmol) in dichloromethane (20ml) was cooled to 0°C, treated with pyridine (489µL, 6mmol) followed by triflic anhydride (929µL, 5.5mmol). The stirred reaction mixture was allowed to warm to room temperature overnight. Water (30ml) was added and the organic layer separated. The aqueous layer was further extracted with dichloromethane (30ml) and the combined organics were dried over sodium sulphate, filtered and evaporated. Purification by flash chromatography on silica using 7% ethyl acetate in isohexane as eluant yielded the title compound (1.6g, 77%). ¹H NMR (400 MHz, CDCl₃) δ 7.38-7.30 (7H, m), 3.72 (3H, s), 3.68 (2H, s), 2.99-2.91 (1H, m), 1.29 (6H, d, *J* = 6.9Hz).

### Step 3: 3-(4-Isopropylphenyl)-4-(4-(trifluoromethyl)phenyl)phenylacetic acid

A 5ml process vial from Personal Chemistry™ was charged with a mixture of methyl (4'-isopropyl-6-{[(trifluoromethyl)sulfonyl]oxy}biphenyl-3-yl)acetate (Step 2, 0.57g, 1.37mmol), 4-(trifluoromethyl)phenyl boronic acid (0.39g, 2.05mmol), [1,1'-diphenylphosphino)ferrocene]dichloropalladium(II), complex with dichloromethane (0.06g, 0.073mmol) and potassium orthophosphate (0.63g, 2.74mmol). The vial was capped and toluene was added to the mixture. The vial was heated for 15 minutes at 170°C, using a Personal Chemistry Smith™ synthesizer and cooled to room temperature. The black mixture was partitioned between water (10ml) and ethyl acetate (50 ml) and the phases were separated. After two more extractions the organic phases were combined, dried over sodium sulphate, filtered and evaporated. Purification by flash chromatography on silica using 5- 10% ethyl acetate in isohexane as eluant yielded methyl 3-(4-isopropylphenyl)-4-(4-(tritluoromethyl)phenyl)phenylacetate (0.32g, 57%) as an oil.

Methyl 3-(4-isopropylphenyl)-4-(4-trifluoromethyl)phenyl)phenylacetate (0.32g, 0.78mmol) was heated at 100°C for 2 hours in 40% aqueous potassium hydroxide solution (10ml). After cooling to room temperature, 1N HCl was added to give a pH of 4 and the aqueous phase was extracted with ethyl acetate (2x20mL). The organic phases were combined, dried over sodium sulphate, filtered and evaporated. Purification by flash chromatography on silica using 10 to 50% ethyl acetate in isohexane as eluant yielded the target compound as a foam which was recrystallised from 70% aqueous methanol to give 280mg (90%) of the target compound as a colourless solid. MS: (ES (M-1)) 397. ¹H NMR (360MHz, CDCl₃) δ 7.45 (2H, d, J = 8.2Hz), 7.38-7.33 (3H, m), 7.25-7.23 (2H, d, *J* = 8.2Hz), 7.08 (2H, d, *J* = 8.0Hz), 7.01 (2H, d, *J* = 8.0Hz), 3.73 (2H, s), 2.85 (1H, sept, J = 6.8Hz), 1.22 (6H, d, J = 6.8Hz).

### Examples 2-26

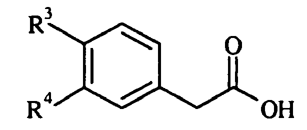

Following the procedure of Example 1, using the appropriate arylboronic acids R⁴-B(OH)₂ and R³-B(OH)₂ in steps 1 and 3 respectively, there were prepared:

| **Example** | **R³** | **R⁴** | **MS (ES)*** |
|---|---|---|---|
| 2 | 2,4-di-CF₃-Ph | 4-isopropyl-Ph | 465 (M-1) |
| 3 | 4-isopropyl-Ph | 4-isopropyl-Ph | 327 |
| 4 | 4-t-butyl-Ph | 4-isopropyl-Ph | 341 |
| 5 | 4-F-Ph | 4-isopropyl-Ph | 303 |
| 6 | 4-Cl-Ph | 4-isopropyl-Ph | 319 |
| 7 | 3-Me-Ph | 4-isopmpyl-Ph | 299 |
| 8 | 4-Me-Ph | 4-isopropyl-Ph | 299 |
| 9 | 2-CF₃-Ph | 4-isopropyl-Ph | 353 |
| 10 | 2,4-di-MeO-Ph | 4-isopropyl-Ph | 345 |
| 11 | 4-(2-Me-propyl)-Ph | 4-isopropyl-Ph | 341 |
| 12 | 2-MeO-5-Me-Ph | 4-isopropyl-Ph | 329 |
| 13 | 5-isoquinolyl | 4-isopropyl-Ph | 336 |
| 14 | 3-CF₃-Ph | 4-isopropyl-Ph | 353 |
| 15 | 4-(4-PhO)-Ph | 4-isopropyl-Ph | 377 |
| 16 | 4-Et-Ph | 4-isopropyl-Ph | 313 |
| 17 | 4-MeO-Ph | 4-isopropyl-Ph | 315 |
| 18 | 4-n-butyl-Ph | 4-isopropyl-Ph | 341 |
| 19 | 2,4-di-Cl-Ph | 4-isopropyl-Ph | 353 |
| 20 | 5-quinolyl | 4-isopropyl-Ph | 336 |
| 21 | 4-isopropoxy-Ph | 4-isopropyl-Ph | 343 |
| 22 | 4-CF₃-Ph | 4-CF₃O-Ph | 395 |
| 23 | 4-CF₃-Ph | 4-n-butyl-Ph | 367 |
| 24 | 2,4-di-CF₃-Ph | 4-n-butyl-Ph | 435 |
| 25 | 4-CF₃-Ph | 4-t-butyl-Ph | 367 |
| 26 | 4-CF₃-Ph | 4-CF₃-Ph | 379 |

| | | | |
|---|---|---|---|
| * (M-1-CO₂) unless otherwise stated. | | | |

### Example 27

### 3,4,5-Tris-(4-(trifluoromethyl)phenyl)phenylacetic acid

Bromine (480mg, 3 mmol) in acetic acid (2ml) was added dropwise to methyl 4-hydroxy-3-(4-(trifluoromethyl)phenyl) acetate (Example 26 Step 1) (930mg, 3mmol) in acetic acid (10ml) and stirred overnight at room temperature. The reaction mixture was evaporated and the residue purified by flash chromatography on silica using 5% ethyl acetate in isohexane as eluant to yield methyl 3-bromo-4-hydroxy-5-(4-(trifluoromethyl)phenyl) acetate (925mg,79%), as a white solid.
The above intermediate was converted to the triflate, reacted with 4-(trinuoromethyl)benzeneboronic acid, and the ester group hydrolysed using the procedures of Example 1 steps 2 and 3 to give the title compound. MS: 523 (ES) (M-1-CO₂).

### Example 28

### 2-(3,4-Bis-(4-(trifluoromethyl)phenyl)nhenyl)-2-methyl-2-propionic acid

Methyl 3,4-bis-(4-(trifluoromethyl)phenyl)phenylacetate (Example 26, 120mg, 0.274rnmol) in tetrahydrofuran (5ml) was cooled to 0°C and treated with potassium tert-butoxide (0.55ml, 1M in THF,0.55mmol). After 20 minutes, iodomethane was added dropwise. The reaction was allowed to warm to room temperature and stirred overnight Water (5ml) was added and the reaction mixture was extracted using ethyl acetate (2 x 20ml). The combined organics were dried over sodium sulphate, filtered and evaporated. Purification by flash chromatography on silica using 5% ethyl acetate in isohexane as eluant yielded the dimethylated ester (90mg). This was hydrolysed by heating in a mixture of 40% potassium hydroxide (2ml)/dioxan (2ml) at 100C for 18 hours. The reaction mixture was cooled to room temperature, acidified using 2N hydrochloric acid and extracted using dichloromethane (30ml). Following evaporation the compound was purified by mass-triggered HPLC to give the title compound (27mg,22%), MS: 407 (ES (M-1-CO₂)).
¹H NMR (500 MHz, CDCl₃) δ 1.69 (6H, s), 7.21-7.26 (4H, m), 7.40-7.44 (2H, m), 7.8-7.54 (5H, m) ppm.

### Example 29

### 2-(3,4-Bis-(4-(trifluoromethyl)phenyl)phenyl)-2-(2-propenyl)-4-pentenoic acid

Prepared as described in Example 28, using allyl bromide in place of methyl iodide. MS: 459 (ES (M-1-CO₂)).

### Example 30

### 3,4-Bis-(4-(trifluoromethyl)phenyl)phenyl-2,2-difluoroacetic acid

Prepared as described in Example 28, using sodium hexamethyldisilazide as the base and N-fluorobenzenesulphonimide as electrophile. MS: 415 (ES (M- 1-CO₂)).

### Example 31

### 1-(3,4-Bis-(4-(trifluoromethyl)phenyl)phenyl)cyclopentane-1-carboxylic acid

Prepared as described in Example 28, using 1,4-diiodobutane in place of methyl iodide. MS: 433 (ES (M-1-CO₂)).

### Example 32

### 2-(3,4-Bis-(4-(trifluoromethyl)phenyl)phenyl)-2-(propyl)pentanoic acid

2-(3,4-Bis-(4-(trifluoromethyl)phenyl)phenyl)-2-(2-propenyl)-4-pentenoic acid (Example 29, 90mg) in ethanol (10ml) was treated with 5% palladium on carbon and hydrogenated using a Parr apparatus at 37 psi for 20 minutes. Filtration and evaporation yielded the title compound as a white solid (60mg, 66%). MS: (ES (M-1-CO₂)) 463.
¹H NMR (500 MHz, CDCl₃) δ 0.94 - 0.97 (6H, tr., J = 7.2 Hz), 1.18 - 1.26 (4H, m), 1.99 - 2.12 (4H, m), 7.22 -7.23 (4H, m), 7.36 -7.51 (7H, m) ppm.

### Example 33

### 2-(3,4-Bis-(4-(trifluoromethyl)phenyl)phenyl)-2-methylpropionic acid

3,4-Bis-(4-(trifluoromethyl)phenyl)phenylacetic acid (Example 28) (170mg, 0.4mmol) in tetrahydrofuran (5ml) was cooled to -78C. The mixture was treated with n-butyl lithium (1.6M in hexanes, 0.65ml, 1mmol), a deep red colour forming on addition of the second equivalent of base. After 20 minutes, iodomethane (32µL, 0.5mmol) was added dropwise. The reaction was allowed to warm to room temperature overnight. Water (5ml) was added, followed by 2N hydrochloric acid (10ml). The reaction mixture was extracted using ethyl acetate (2x20ml), dried over sodium sulphate, filtered and evaporated. The residue was purified by mass-triggered HPLC to give the title compound (42mg, 24%).
¹H NMR (500 MHz, CDCl₃) δ 1.6 (3H, d, J = 7.2 Hz); 3.86 (1H, quart., J = 7.2 Hz), 7.20 -7.26 (4H, m), 7.38 - 7.50 (7H, m) ppm.

### Example 34

### 2-(3,4-Bis-(4-(trifluoromethyl)phenyl)phenyl)hexanoic acid

Prepared as described in Example 33, using iodobutane in place of iodomethane. MS: 435 (ES (M-1-CO₂)).

### Example 35

### 2-(3,4-Bis-(4-(trifluoromethyl)phenyl))phenyl)-3-phenylpropionic acid

Prepared as described in Example 33, using benzyl bromide in place of iodomethane., MS: 469 (ES (M-1-CO₂)).

### Example 36

### 4-(Phenylethynyl)-3-(4-isopropylphenyl)phenylacetic acid

Methyl (4'-isopropyl-6- {[(trifluoromethyl)sulfonyl]oxy}biphenyl-3- y1)acetate (Example I Step 2), (416mg, 1mmol), phenylacetylene (204mg, 2mmol), tetrakis(triphenylphosphine)palladium(0) 58mg, 0.05mmol), triphenylphosphine (26.2mg, 0.1mmol) and copper (I) iodide (19.5mg, 0.1 mmol) in triethylamine (2ml) were heated at 180°C for 10minutes using a Personal Chemistry Smith synthesizer and cooled to room temperature. The reaction mixture was partitioned between ethyl acetate(20ml) and water (30ml). The organic layer was washed with 2N hydrochloric acid (30ml) and brine (30ml), dried over sodium sulphate, filtered and evaporated. Following flash chromatography on silica using 1% ethyl acetate in isohexane as eluant, the residue was dissolved in dioxan (3ml) and treated with lithium hydroxide (100mg) in water (3ml) and stirred at room temperature overnight. The reaction was acidified using 2N hydrochloric acid (5ml) and extracted using ethyl acetate (2x10ml), dried over sodium sulphate, filtered and evaporated. The residue was purified by mass-triggered HPLC to give the title compound (20mg, 6%), MS: 309 (ES (M-1-CO₂)).
¹H NMR (500 MHz, CDCl₃) δ 1.31 (6H, d, J = 7.0 Hz), 2.98 (1H, sept., J = 7.0 Hz), 3.72 (2H, s), 7.26 - 7.39 (7H, m), 7.52 - 7.62 (5H, m) ppm.

### Example 37

### 4-(4-(Trifluoromethyl)phenylethynyl)-3-(4-isopropylphenyl)phenylacetic acid

Pepared as described in Example 36, using 4-(trifluoromethyl)phenylacetylene in place of phenylacetylene. MS: 377 (ES (M-1-CO₂)).

### Example 38

### 3-(4-isopropylphenyl)-4-(4-trifluoromethylphenoxy)phenylacetic acid

Methyl (4-hydroxy-3-(4-isopropylphenyl))phenyl acetate (Example 1, Step 1, 1.42g, 5mmol), activated molecular sieve powder (1.5g), copper (II) acetate(908mg, 5mmol) and 4-trifluoromethylbenzene boronic acid in dichloromethane (50ml) was treated with triethylamine. The reaction mixture was stirred overnight at room temperature, filtered and evaporated. Purification by flash chromatography on silica using 1-5% ethyl acetate in isohexane as eluant yielded methyl 3-(4-isopropylphenyl)-4-(4-trifluoromethylphenoxy)phenylacetate (140mg). This ester (100mg) was dissolved in a mixture of 40% potassium hydroxide solution (1ml) and 1,4-dioxan(2ml), heated at 100°C for 18 hours, cooled to room temperature, acidified using 2N hydrochloric acid and extracted using dichloromethane to give the title compound (78mg). MS: (ES (M-1)) 413.
¹H-NMR (500MHz CDCl3) d: (ppm)) 7.48 (d, 2H, J = 8.7 Hz), 7.41-7.37 (m, 3H), 7.27-7.24 (m, 1H) 7.19 (d, 2H, J = 8.2 Hz), 7.01 (d, 1H, J = 8.3 Hz), 6.95 (d, 2H, J = 8.6 Hz), 3.71 (s, 2H), 2.91-2.85 (m, 1H), 1.23 (d, 6H, J = 6.9 Hz).

### Example 39

### 3-(4-isopropylphenyl)-4-(4-trifluoromethylbenzyloxy)phenylacetic acid

Methyl (4-hydroxy-3-(4-isopropylphenyl))phenyl acetate (Example 1, Step 1, 569mg, 2mmol) in DMF (10ml) was treated with potassium carbonate (553mg, 4mmol). After 10 minutes 4-tritluoromethylbenzyl bromide in DMF (1ml) was added. After stirring for 18 hours, the solvent was evaporated, and the residue partitioned between water (30ml) and ethyl acetate (50ml). The aqueous layer was extracted with further ethyl acetate (50ml). The combined organics were dried over sodium sulphate, filtered and evaporated. Purification by flash chromatography on silica using 5% ethyl acetate in isohexane as eluant yielded methyl 3-(4-isopropylphenyl)-4-(4-trifluoromethylbenzyloxy)phenylacetate (670mg). This ester (200mg)) was dissolved in a mixture of 40% potassium hydroxide solution (1.5ml) and 1,4-dioxan (0.75ml), heated at 100°C for 18 hours, cooled to room temperature, acidified using 2N hydrochloric acid and extracted using dichloromethane. Crystallisation from ethyl acetate:isohexane gave the title compound (50mg). MS: (ES (M-1)) 413.
¹H NMR (500MHz CDCl₃) d (ppm) : 7.56 (2 H, d, J = 8.0 Hz), 7.48 (2 H, d, J = 8.0 Hz), 7.41 (2 H, d, J = 8.0 Hz), 7.3- 7.24 (3 H, m); 7.19 (1 H, d, J = 8.3 Hz), 6.94 (1 H, d, J = 8.3 Hz), 5.11 (2 H, s), 3.64 (2 H, s), 2.99-2.93 (1 H, m), 1.30 (6 H, d, J = 6.8 Hz).

### Example 40

### 4-(4-Methyl-1-pentyloxy)-3-(4-(trifluoromethyl)phenyl)phenylacetic acid

Prepared by the method of Example 39, but using 4-methyl- 1-pentyt iodide in place of 4-trifluoromethylbenzyl bromide. MS: 379 (ES (M-1-CO₂)).

### Example 41

### 4-(1-(4-Phenyl)piperazinyl)-3-(4-(tritluoromethyl)phenyl)phenylacetic acid

3-Bromo-4-fluoroacetophenone (4.34g, 20 mmol) in dioxan (60ml) was treated with 4-trifluoromethylphenyl boronic acid (5.3g, 30mmol) and saturated sodium carbonate solution (30ml). After degassing with nitrogen , [1,1'-diphenylphosphino)ferrocene]-dichloropaHadium(II), (816mg, 1 mmol) was added and the mixture degassed. The reaction was heated for 18 hours at 100°C, cooled to room temperature and water (50ml) added. The reaction mixture was extracted with ethyl acetate (4x50ml). The combined organics were dried over sodium sulphate, filtered and evaporated. Purification by flash chromatography on silica using 5% ethyl acetate in isohexane as eluant yielded 3-(4-(trifluoromethyl)phenyl-4-fluoroacetophenone (4.6g, 81%). This intermediate (847mg, 3mmol), potassium carbonate (415mg, 3mmol) and phenylpiperazine (383mg, 3.6mmol) in DMSO (3ml) were heated at 95°C for 18 hours. The mixture was partitioned between water (15ml) and ethyl acetate (35ml). The organic layer was washed with brine (15ml), dried over sodium sulphate, filtered and evaporated. Purification by flash chromatography on silica using 10% ethyl acetate in isohexane as eluant yielded 3-(4-(trifluoromethyl)phenyl)-4-(1-(4-phenyl)piperanyl)-acetophenone (160mg, 13%).
This compound (141m, 0.33mmol) was treated with sulphur (27mg, 0.83mmol) and morpholine (174uL, 2mmol) and the mixture heated at 170°C for 10 minutes using an Emrys Optimiser microwave synthesizer, then cooled to room temperature. The crude mixture was purified by flash chromatography on silica using 20% ethyl acetate in isohexane as eluant to give 4- {2-[6-(4-phenylpiperazin-1-yl)-4'-(trifluoromethyl)biphenyl-3-yl]ethanethioyl}morpholine. (142mg,81 %).
This thioamide (50mg, 0.095mmol) in ethanol (15ml) was treated with 4N sodium hydroxide and heated at reflux overnight. The solvent was removed and the residue was treated with 2N hydrochloric acid. Water (5ml) was added and the aqueous was extracted using dichloromethane containing methanol. The combined organics were evaporated and purified by mass-triggered HPLC to give the title compound (7.5 mg, 14%) as the trifluoroacetate salt, MS: 441 (ES (M+1)).
¹H NMR (500 MHz, CDCl₃) δ 3.25 (4H, m, br.), 3.39 (4H, m, br.), 3.66 (2H, s), 7.12 - 7.52 (8H, m), 7.68 - 7.73 (4H, m) ppm.

### Example 42

### 3,4-Bis-(4-(trifluoromethyl)phenyl)benzoic acid

A 5 ml process vial from Personal Chemistry was charged with a mixture of 3,4-dichlorobenzoic acid (191mg, 1mmol), 4-(trifluoromethyl)phenyl boronic acid (0.57g, 3mmol), [1,1'-diphenylphosphino)ferrocene]dichloropalladium(II), (41mg,, 0.05mmol) and potassium orthophosphate (848mg, 4 mmol). The vial was capped and toluene was added to the mixture. The vial was heated for 15 minutes at 170°C, using a Personal Chemistry Smith synthesizer and cooled to room temperature. The mixture was filtered through Hyflo and evaporated. The compound was purified by mass-triggered HPLC to give the title compound (11.8mg, 3%), MS: 409 (ES (M-1)).
¹H NMR (500 MHz, CDCl₃) δ 7.26-7.27 (4H, m), 7.53-7.56 (5H, m), 8.18-8.21 (2H, m) ppm.

### Example 43

### 3-(4-Isopropylphenyl)-4-(4-(trifluoromethyl)phenyl)phenylpropionic acid

Prepared using the procedure of Example 1 but starting with methyl 3-(3-bromo-4-hydroxyphenyl)propionate. Colourless solid, MS: 411 (ES (M-1)).
¹H NMR (400 MHz, CDCl₃): d 7.45 (d, 2 H J = 8.2Hz), 7.22-7.33 (m, 5 H), 7.08 (d, 2 H, J = 8.0Hz), 7.02-7.00 (d, 2 H, J = 8.0Hz), 3.05 (t, 2 H, J = 7.7 Hz), 2.90-2.81 (m, 1 H), 2.77 (t, 2 H, J = 7.7 Hz), 1.23 (d, 6 H J = 6.9 Hz) ppm.

### Example 44

### 2,3-Bis-(4-(trifluoromethyl)phenyl)pyridyl-6-acetic acid

### Step 1- 2,3-bis-(4-(trifluoromethyl)phenyl)-6-methylpyridine

Prepared as described in Example 1 Step 1 using 3-bromo-2-chloro-6-methyl pyridine and 4-(trifluoromethyl)benzeneboronic acid. MS: 382 (ES (M+1)).
¹H NMR (360 MHz, CDCl₃) δ 2.68 (3H, s), 7.25-7.28 (3H, m), 7.45 (2H, d, J = 8.2 Hz), 7.51-7.55 (4H, m), 7.64 (1H, d, J = 7.9 Hz) ppm.

### Step 2

n-Butyllithium (7.5 mL of 1.6 M solution in hexane, 12mmol) was added dropwise to a solution of diisopropylamine (1.76 mL, 12.6 mmol) in anhydrous THF (10 mL) at -20°C under an atmosphere of nitrogen. After stirring for 5 minutes the solution was cooled to - 78°C and 2,3-bis-(4-(trifluoromethyl)phenyl)-6-methylpyridine (Step 1) (2.18 g, 5.7 mmol, dissolved in 2 mL of THF) was added over 5 minutes. The deep blue solution was stirred for 1 hour and dimethylcarbonate (0.97 mL, 11.4 mmol) was added over 1 minute. The cool bath was removed and stirring was continued at 0°C for another hour, before the reaction was quenched by adding saturated aqueous ammonium chloride (10 mL) and ethyl acetate (20 mL). The phases were separated and the aqueous phase was extracted twice. The combined organic phases were washed with water and brine, dried over sodium sulphate, filtered and evaporated. Purification by flash chromatography on silica using 5-10% ethyl acetate in isohexane as eluant yielded 1.4 g (56 %) of methyl 2,3-bis-(4-(trifluoromethyl)phenyl)pyridyl-6-acetate as a yellow oil, MS: 439 (ES (M+1)).
This methyl ester (0.28 g, 0.64 mmol) was dissolved in THF (4 mL) and water (2 mL) followed by lithium hydroxide (70 mg; 2.92 mmol) were sequentially added. The mixture was heated at 70 °C for 15 hours, allowed to cool to room temperature and poured into a mixture of ethyl acetate/ 1 N HCl (20 ml/ 4 ml). The phases were separated and the aqueous phase was extracted two times with ethyl acetate (20 ml each). The combined organic layers were washed with brine, dried over sodium sulfate and adsorbed onto silica gel. Purification by flash chromatography (50 % ethyl acetate in iso-hexane) yielded 0.22 g (81 %) of the target compound as a colourless solid, MS: 426 (ES (M+1)).
¹H NMR (360 MHz, DMSO) δ 3.54 (2H, s), 7.40 (2H, d, J = 8.1 Hz), 7.47-7.50 (3H, m), 7.64-7.70 (4H, m), 7.77 (1H, d, J = 8.0 Hz) ppm.

### Example 45

### 2,3-Bis-(4-(LI-ifluoromethyl)phenyl)pyridyl-5-acetic acid

Prepared as described in Example 44, starting from 3-bromo-2-chloro-5-methyl pyridine and using 1,3-dimethyl-3,4,5,6-tetrahydro-2(1H)-pyrimidinone (2 equivalents) as a cosolvent in the deprotonation step. Colourless solid, MS: 426 (ES (M+1)).
¹H NMR (360 MHz, CDCl₃) δ 3.76 (2H, s), 7.27 (2H, d, J = 7.4 Hz), 7.42 (2H, d, J = 8.2 Hz), 7.51-7.57 (4H, m), 7.73 (1H, d, J = 1.9 Hz), 8.71 (1H, d, J = 1.9 Hz) ppm.

Examples 46-54 were prepared by the procedure of Example 33 using the appropriate iodoalkane derivative in place of iodomethane and lithium diisopropylamide in place of butyl lithium.

### Example 46

### 2-(3,4-Bis-(4-(trifluoromethyl)phenyl)phenyl)hexanoic acid

MS: 435 (ES (M-1-CO₂)).

### Example 47

### 2-(3,4-Bis-(4-(trifluoromethylphenyl)phenyl)butanoic acid

MS: 407 (ES (M-1-CO₂)).

### Example 48

### 2-(3,4-Bis-(4-(trifluoromethyl)phenyl)phenyl)pentanoic acid

MS: 421 (ES (M-1-CO₂)).

### Example 49

### 2-(3,4- Bis-(4-(trifluoromethyl)phenyl)phenyl)-3-methylbutanoic acid

MS: 421 (ES (M-1-CO₂)).

### Example 50

### 2-(3,4-Bis-(4-(trifluoromethyl)phenyl)phenyl)-4-methylpentanoic acid

MS: 435 (ES (M-1-CO₂)).

### Example 51

### 2-(3,4-Bis-(4-(trifluoromethyl)phenyl)phenyl)-3-cyclopropylproic acid

MS: 433 (ES (M-1-CO₂)).

### Example 52

### 2-(3,4-Bis-(4-(trifluoromethyl)phenyl)phenyl)-5,5,5 trifluoropentanoic acid

MS: 475 (ES (M-1-CO₂)).

### Example 53

### 2-(3,4-Bis-(4-(trifluoromethyl)phenyl)phenyl)-6-chlorohexanoic acid

MS: 513 (ES (M-1)).

### Example 54

### 2-(3,4-Bis-(4-(trifluoromethyl)phenyl)phenyl)-4-methylpent-4-enoic acid

MS: 433 (ES (M-1-CO₂)).

### Example 55

### 3-(4-(Trifluoromethyl)phenyl)-4-(2,4-bis(trifluoromethyl)phenyl)phenylacetic acid

Prepared by the procedure of Example 1 using 4-(trifluoromethyl)phenyl boronic acid in Step I and 2,4-bis(trifluoromethyl)phenyl boronic acid in Step 3
MS: 447 (ES (M-1-CO₂)).

### Example 56

### 3-(4-(Trifluoromethyl)phenyl)-4-(2-chloro-4-(trifluoromethyl)phenyl)phenylacetic acid

Prepared by the procedure of Example 1 using 4-(trifluoromethyl)phenyl boronic acid in Step 1 and 2-chloro-4-(trifluoromethyl)phenyl boronic acid in Step 3.

### Example 57

### 3-(2,4-Bis(trifluoromethyl)phyhenyl)-4-(4-(trifluoromethyl)phenyl)phenylacetic acid

### Step 1

### 3-bromo-4-(4-(trifluoromethyl)phenyl)toluene

To 3-bromo-4-iodotoluene (14g ; 47.15mmol) and 4-(trifluoromethyl)benzeneboronic acid (9.85g ; 51.8mmol) in 1,4-dioxan (100ml) and saturated sodium carbonate solution (40ml) under nitrogen was added [1,1'-bis(diphenylphosphino)ferrocene]palladium(II) chloride (1.73g ; 2.36mmol). The reaction mixture was heated at 80°C for 18h, diluted with water and ethyl acetate and filtered through hyflo. The filtrate was separated, the organic extracts were combined, washed with brine, dried over magnesium sulphate filtered and evaporated under reduced pressure to give an oil. The oil was absorbed onto silica and purified by flash chromatography using iso-hexane as eluant. The appropriate fractions were combined and evaporated under reduced pressure to give the title compound.

### Step 2

### methyl 3-bromo-4-(4-(trifluoromethyl)phenyl)phenylacetate

To a solution of diisopropylamine (6.4221g 8.944ml ; 63.4662mmol) in THF (100ml) at - 20°C was added a solution of 1.6M n-butyllithium (39.6mls 63.47mmol). On completion of the addition the reaction mixture was stirred for ten minutes then cooled to -78°C. 1,3-Dimethyl-3,4,5,6-tetrahydro-2(1H)-pyrimidinonc (8.13g 7.675ml; 63.47mmol) was added followed by a solution of 3-bromo-4-(4-(trifluoromethyl)phenyl)toluene (7.6g; 24.1172mmol) in THF dropwise. After stirring for an hour at -78°C, dimethyl carbonate (4.345g 4.061 ml; 48.2344mmol) was added drop wise. On completion of the addition the reaction mixture was allowed to warm to 0°C and to stir for an hour. The reaction mixture was quenched by the addition of a solution of ammonium chloride, extracted with ethyl acetate, and the combined ethyl acetate extracts washed with 2N hydrochloric acid, brine, dried over magnesium sulphate, filtered, then evaporated under reduced pressure to give an oil. The oil was purified by flash chromatography using iso-hexane-iso-hexane/ethyl acetate (10:1) as eluant. The appropriate fractions were combined and evaporated under reduced pressure to give the title compound.

### Step 3

### Methyl 3-(2,4-Bis(trifluoromethyl)phenyl)-4-(4-(trifluoromethyl)phenyl)phenylacetate

To a solution of methyl 3-bromo-4-(4-(trifluoromethyl)phenyl)phenylacetate (.5g; 1.34mmol) and 2,4-bis(trifluoromethyl)benzeneboronic acid (.52g; 2.00mmol) in 1,4-dioxan (4ml) and saturated sodium carbonate solution (1ml) was added [1,1'-bis(diphenylphosphino)ferrocene]palladium(II) chloride (0.049g ; 0.067mmol). The reaction mixture was heated in a microwave reactor at 170°C for fifteen minutes then poured onto water and extracted with ethyl acetate. The ethyl acetate extracts were combined, washed with brine, dried over magnesium sulphate filtered and evaporated under reduced pressure to give an oil. The oil was absorbed onto silica and purified by flash chromatography using a gradient elution iso-hexane to iso-hexane/ethyl acetate (10:1) as eluant. The appropriate fractions were combined and evaporated under reduced pressure to give the title compound Yield = 0.5g

### Step 4

### 3-(2,4-Bis(trifluoromethyl)phenyl)-4-(4-(trifluoromethyl)phenyl)phenylacetic acid

Prepared by hydrolysis of the ester from Step 3 under the conditions described in Example 1 Step 3.
¹H NMR (400 MHz, CDCl₃) δ 3.75 (2H, s), 7.17 - 7.20 (3H, m), 7.26(2H, m) 7.41-7.48 (4H, m), 7.60 (1H, d, J= 8.7 Hz) 7.94 (1H, s) ppm.
MS: (ES (M-H-CO2) = 447

### Example 58

### 3-(2-Chloro-4-(trifluoromethyl)phenyl)-4-(4-(trifluoromethyl)phenyl)phenylacetic acid

The product of Example 57 Step 2 was hydrolysed as described under Example 1 Step 3. The resulting acid (0.2g ; 0.557mmol) and 2-chloro-4-(trifluoromethyl)benzene boronic acid (0.19g ; 0.84mmol) and [1,1'-bis(diphenylphosphino)ferrocene]palladium(II) chloride (0.0407g ; 0.05569mmol) in 1,4-dioxan (4ml) and saturated sodium carbonate (1ml) were heated at 170°C for fifteen minutes in a microwave reactor. The reaction mixture was diluted with dichloromethane and passed through a phase separation cartridge. The dichloromethane extracts were combined, evaporated under reduced pressure and the residue absorbed onto silica and purified by flash chromatography using iso-hexane-ethyl acetate gradient elution. The appropriate fractions were combined and evaporated under reduced pressure to give a solid. The solid was triturated with acetonitrile and collected by filtration and dried Yield = 38mgs.
¹H NMR (400 MHz, CDCl₃) δ 3.76 (2H, s), 7.20 - 7.27 (5H, m), 7.40-7.48 (5H, m), 7.60 (1H, s) ppm.
MS: (ES (M-H-CO₂) =413

### Example 59

### 2-(3-(4-(Trifluoromethyl)phenyl)-4-(2,4-bis(trifluoromethyl)phenyl)phenyl)-4-methylpentanoic acid

Prepared from the product of Example 55 by the process described in Example 33, using 1-iodo-2-methylpropane in place of iodomethane and lithium diisopropylamide as base in place of butyl lithium.
MS: 503 (ES (M-1-CO₂)).

### Example 60

### 2-(3-(4-(Trifluoromethyl)phenyl)-4-(2-chloro-4-(trifluoromethyl)phenyl)phenyl)-4-methylpentanoic acid

Prepared from the product of Example 56 by the process described in Example 33, using 1-iodo-2-methylpropane in place of iodomethane and lithium diisopropylamide as base in place of butyl lithium.
MS: 469 (ES (M-1-CO₂)).

### Example 61

### 2-(3-(4-(Trifluoromethyl)phenyl)-4-(2-chloro-4-(trifluoromethyl)phenyl)phenyl)-4-methylpent-4-enoic acid

Prepared from the product of Example 56 by the process described in Example 33, using 3-bromo-2-methylpropene in place of iodomethane and lithium diisopropylamide as base in place of butyl lithium. MS: 467 (ES (M-1-CO₂)).

### Example 62

### 2-(3-(2,4-Bis(trifluoromethyl)phenyl)-4-(4-(trifluoromethyl)phenyl)phenyl)pentanoic acid

Prepared from the product of Example 57 by the process described in Example 33, using bromopropane in place of iodomethane.
MS: (ES (M-H-CO₂) = 489

### Example 63

### 3-(2,4-Bis(trifluoromethyl)phenyl)-4-(4-(trifluoromethyl)phenyl)phenyl)-4-methyl-2-pentanoic acid

Prepared from the product of Example 57 by the process described in Example 33, using 1-bromo-3-methylbutane in place of iodomethane.
MS: (ES (M-H-CO₂) = 503

### Example 64

### 3-(2,4-Bis(trifluoromethyl)phenyl)-4-(4-(trifluoromethyl)phenyl)phenyl)cyclopentane-1-carboxylic acid

Prepared from the product of Example 57 by the process described in Example 28, using 1,4-diiodobutane in place of methyl iodide.
MS: (ES (M-H-CO₂) = 501

### Example 65

### α- Isopropoxy- 3,4-bis(4-(trifluoromethyl)phenyl)phenylacetic acid

### Step 1

### methyl α-diazo-(3,4-bis-(4-(trifluoromethyl)phenyl)phenyl)acetate

To methyl 3,4-bis(4-(trifluoromethyl)phenyl)phenylacetate (2g; 4.5625mmol) and 4-acetamidobenzenesulfonyl azide (1.0961g; 4.5625mmol) in acetonitrile (10ml) cooled to - 10°C in an ice/salt bath was added 1,8-diazabicyclo[5.4.0]undec-7-ene (0.7641g 0.749ml; 5.01875mmol) dropwise. The reaction mixture was allowed to warm up to room temperature and stir overnight. The solvent was evaporated under reduced pressure and the residue partitioned between diethyl ether and sodium carbonate solution. The diethyl ether extracts were combined washed with brine, dried over magnesium sulphate, filtered and evaporated under reduced pressure to give an oil. The oil was purified by flash chromatograhy using iso-hexane/ethyl acetate (25:1) as eluant. The appropriate fractions were combined and evaporated under reduced pressure to give a solid.

### Step 2

### α-isopropoxy-3,4-bis-(4-(trifluoromethyl)phenyl)phenylacetic acid

To a solution of isopropanol and rhodium(II) acetate dimer dihydrate (46.3273mg ; 0.096915mmol) at reflux was added a solution of the diazo derivative from Step 1 (0.3g; 0.6461mmol) in toluene dropwise. The reaction mixture was refluxed for a further fifteen minutes, then sodium hydroxide was added and the reaction mixture heated at reflux for a further 2h. The solvent was evaporated under reduced pressure and the residue acidified with 2N HCl. The solution was extracted with ethyl acetate, and the combined extracts washed with brine dried, over magnesium sulphate, filtered and evaporated under reduced pressure to give an oil. The oil was dissolved in DMSO and purified by mass-directed HPLC. The appropriate fractions were combined and evaporated under reduced pressure to give an oil. The oil was extracted with dichloromethane, the dichloromethane extracts were combined dried over magnesium sulphate, filtered and evaporated under reduced pressure to give an oil which on trituration with hexane gave a solid. The solid was collected by filtration washed with hexane and dried to give the title compound.
¹H NMR (400 MHz, DMSOd₆) δ 1.65 (6H, m) 3.70 (1H, m), 7.31-7.35 (4H, m), 7.46-7.51 (2H, m), 7.57-7.65 (5H, m) ppm.
MS: (ES (M-H) = 481

### Example 66

### 1-(3,4-Bis-(4-(trifluoromethyl)phenyl)phenyl)cyclobutane-1-carboxylic acid

### Step 1

### 1-fluoro-(3,4-bis-(4-(trifluoromethyl)phenyl)benzene

2-Bromo-4-fluoroiodobenzene (15.5g, 51.5mmol) in dioxan (150ml) was treated with 4-(trifluoromethyl)benzene boronic acid (28.5g, 150mmol) followed by saturated sodium carbonate solution (100ml). The reaction mixture was degassed with nitrogen, [1,1'-diphenylphosphino)ferrocene]dichloropalladium(II) (2.04g, 2.5mmol) was added and the reaction mixture degassed. The reaction was heated at 100 °C for 18 hours, cooled to room temperature and water (50ml) was added. The reaction mixture was filtered through Hyflo and extracted using ethyl acetate (4x100ml). The combined organics were dried over sodium sulphate, filtered and evaporated. Purification by flash chromatography on silica using 0-2% ethyl acetate in isohexane as eluant yielded 1-fluoro-(3,4-bis-(4-(trifluoromethyl)phenyl)benzene (17.2g, 87%).

### Step 2

### 1-(3,4-bis(4-(trifluoromethyl)phenyl)phenyl)cyclobutane-1-carbonitrile

Potassium hexamethyldisilazide (30ml, 0.5M in toluene, 15mmol) was added dropwise to a mixture of 1-fluoro-(3,4-bis(4-(trifluoromethyl)phenyl)benzene (3.71g, 9.65mmol) and cyclobutanecarbonitrile (3.13g, 39mmol) in toluene (10ml) at ambient temperature. The mixture was heated at 85 °C for 18 hours. Water (50ml) was added and the mixture was extracted using ethyl acetate (4x50ml). The combined organics were dried over sodium sulphate, filtered and evaporated. Purification by flash chromatography on silica using 5-10% ethyl acetate in isohexane as eluant yielded 1-(3,4-bis(4-(trifluoromethyl)phenyl)phenyl)cyclobutane-1-carbonitrile (2.3g).

### Step 3

1-(3,4- Bis(4-(trifluoromethyl)phenyl)phenyl)cyclobutane-1-carbonitrile (1.2g) and potassium hydroxide (2g) in water (2ml)/ethanol(18ml) were heated at 95 °C for 18 hours. The reaction mixture was evaporated, acidified using hydrochloric acid (5N) and extracted using ethyl acetate. The combined organics were dried over sodium sulphate, filtered and evaporated. Purification by mass-triggered HPLC gave the title compound (460mg). ¹H NMR (500 MHz, CDCl₃): d 1.92-2.00 (1H, m), 2.12-2.20 (1H, m), 2.28-2.65 (2H, m), 2.90-2.96 (2H, m), 7.21-7.26 (4H, m), 7.36 (1H, s), 7.40-7.46 (2H, m), 7.49 (4H, dd, J =3.8, 8.0Hz) ppm.
MS: 419 (ES (M-1-CO₂)).

### Example 67

### 1-(3,4-Bis(4-(trifluoromethyl)phenyl)phenyl)-3-methylcyclobutane-1-carboxylic acid (syn and anti-isomers)

Prepared as in Example 66 using 3-methylcyclobutanecarbonitrile in place of cyclobutanecarbonitrile. Both syn and anti isomers were obtained.
MS: 433 (ES (M-1-CO₂)).

### Example 68

### 3-(4-(Trifluoromethyl)phenyl)-4-(2-propyl-4-(trifluoromethyl)phenyl)phenylacetic acid

### Step 1

### Ethyl 4-amino-3-iodophenylacetate

Ethyl 4-aminophenylacetate (21.2 g, 0.12 mol) was dissolved in acetonitrile (100 ml) followed by the addition of sodium hydrogen carbonate (20g, 0.24 mol) and water (200 ml). The mixture was cooled to 0°C and iodine (31 g, 0.12 mol) was added in 5 portions over 30 seconds whilst stirring vigorously. The black reaction mixture was strirred for an additional 15 hours whilst warming up to room temperature. Water was added and the mixture was extracted using diethyl ether (5x 100ml). The combined organic extracts were washed with water, saturated sodium thiosulfate solution and brine and dried over sodium sulphate, filtered and adsorbed onto silica gel. Purification by flash chromatography on silica gel using 5-10% ethyl acetate in isohexane as eluant yielded the title compound as a red oil (21.5 g, 59%).

### Step 2.

### Ethyl 4-amino-3-(4-(trifluoromethyl)phenyl)phenylacetate

Ethyl 4-amino-3-iodophenylacetate (2.72 g, 8.9 mmol), 4-(trifluoromethyl)phenyl boronic acid (2.54 g, 13.3 mmol), [1,1'-diphenylphosphino)ferrocene]-dichloropalladium(II), complex with dichloromethane (0.37 g, 0.45 mmol) and 2M aqueous sodium carbonate solution (9 ml, 18 mmol) were added to 20 ml dioxane. The flask was degassed, heated at 100°C for 12 hours and cooled to room temperature. The black mixture was partitioned between water (50 ml) and ethyl acetate (100 ml) and the phases were separated. After two more extractions the organic phases were combined, dried over sodium sulphate, filtered and evaporated. Purification by flash chromatography on silica using 5-10% ethyl acetate in isohexane as eluant yielded the target compound as an oil.

### Step 3

### Ethyl 4-iodo-3-(4-(trifluoromethyl)phenyl)phenylacetate

Ethyl 4-amino-3-(4-(trifluoromethyl)phenyl)phenylacetate (53.1 g, 0.164 mol) was dissolved in 400 ml of diethyl ether and hydrochloric acid (60 ml of 4 M solution in dioxane, 0.24 mol) was added at room temperature. The reaction was left for 15 minutes to cool to room temperature and iso-hexane was added until the solution became turbid. The mixture was left to crystallize for 1 hour and the obtained solid was filtered, washed with iso-hexane and dried on the sinter to furnish 55 g (93 %) of a light rosé solid. The obtained hydrochloride (27.8 g, 77.4 mmol) was suspended in 300 ml of concentrated hydrochloric acid and cooled to 0 °C. A solution of sodium nitrite (5.4 g, 78 mmol) in water (40 ml) was added dropwise and the heterogeneous reaction was left for an additional 10 minutes at the same temperature. Then a solution of potassium iodide (38 g, 0.23 mol) in water (150 ml) was added dropwise whilst the temperature was kept at 0 °C. The temperature was raised to room temperature and the black mixture was left to stir overnight and finally quenched by pouring it onto ice-water. The product was extracted with ethyl acetate (3 x 100 ml) and the combined organic phases were dried over sodium sulphate, filtered and evaporated. Purification by flash chromatography on silica using 5-10% ethyl acetate in isohexane as eluant yielded the target compound as an oil (27g, 80 %).

### Step 4

### 4-Iodo-3-(4-trifluoromethylphenyl)phenylacetic acid

Hydrolysis of the ester of Step 3 using the procedure described in Example 1 Step 3 and purification by flash chromatography on silica using 100% ethyl acetate as eluant gave the target compound as a yellow solid (6.0 g, 19 %).
¹H NMR (400 MHz, CDC13) δ 3.63 (2H ,s), 7.01 (1H, dd, J = 8.1, 1.9 Hz), 7.19 (1H, d, J = 1.9 Hz), 7.44 (2H, d, J = 7.8 Hz), 7.70 (2H, d, J = 7.8 Hz), 7.91 (1H, d, J = 8.1 Hz) ppm.

### Step 5

### 2-(1-Propyl)-4-trifluoromethylphenylboronic acid pinacol ester

4-Bromobenzotrifluoride (35 ml, 0.25 mol) was dissolved in 100 ml of triflic acid at room temperature. N-iodosuccinimide (59 g, 0.26 mol) was added portionwise over 5 minutes and the dark mixture was stirred for an additional 15 hours at room temperature. The reaction mixture was put onto ice (300 g) and after the ice had melted, the aqueous phase was extracted with 3 portions (100 ml) of diethyl ether. The organic phases were combined, washed with saturated sodium carbonate, water and brine and dried over magnesium sulfate. After filtration and evaporation of the solvent, the remaining liquid was distilled under vacuum (0.25 mbar) to yield 4-bromo-3-iodobenzotrifluoride (85 g, 85 %) as a clear liquid, boiling point: 62-64 °C.
n-Propylmagnesium chloride (50 ml of a 2 M solution in THF, 0.1 mol) was added at room temperature under nitrogen to 200 ml of zinc chloride (0.5 M solution in THF, 0.1 mol). The suspension was heated at 50 °C for 4 hours and cooled to room temperature again. 4-Bromo-3-iodobenzotrifluoride (35 g, 0.1 mol), [1,1'-diphenylphosphino) ferrocene]dichloropalladium(II), complex with dichloromethane (3.66 g, 5 mmol) and copper(I) iodide (1.12 g, 5.8 mmol) were added at room temperature and the reaction was stirred for an additional 3 hours. Water was added and the mixture was neutralized using 2N hydrochloric acid and extracted using diethyl ether (5x 100ml). The combined organic extracts were dried over sodium sulphate, filtered and evaporated. The remaining liquid was distilled under vacuum (57 mbar) to yield a 4: 1 mixture of 4-bromo-3-(1-propyl)benzotrifluoride and the starting material (15 g) as a clear liquid, Boiling point: 112-114°C.
4-Bromo-3-(1-propyl)benzotrifluoride (3.55 g, 13.3 mmol) was dissolved in THF (30 ml) under nitrogen and cooled to -75 °C. n-Butyl lithium (9.1 ml of 1.6 M in hexane, 14.5 mmol) was added dropwise over 1 minute and the resulting orange solution was stirred for 5 minutes at the same temperature. 2-Propoxy pinacol boronate (3 ml, 14.6 mmol) was added dropwise and the resulting solution was warmed up to room temperature and stirred for an additional 3 hours. Water was added and the mixture was extracted using diethyl ether (3x20ml). The combined organic extracts were dried over sodium sulphate, filtered and evaporated to yield the title compound (4 g) as an orange syrup which was used as such in the next step.
¹H NMR (500 MHz, CDCl3) δ 0.95 (3H, t, J = 7.3 Hz), 1.35 (12H, s), 1.58 - 1.59 (2H, m), 2.89 (2H, t, J = 7.7 Hz), 7.39 - 7.40 (2H, m), 7.84 (1H, d, J = 8.3 Hz) ppm.

### Step 6

### 3-(4-(Trifluoromethyl)phenyl)-4-(2-propyl-4-(trifluoromethyl)phenyl)phenylacetic acid

A 5ml process vial from Personal Chemistry™ was charged with a mixture of the product of Step 4 (406mg, 1mmol), the product of Step 5 (628mg, 2mmol), [1,1'-diphenylphosphino)ferrocene]-dichloropalladium(II), complex with dichloromethane (0.041g, 0.05mmol), dioxan (2ml) and saturated sodium carbonate solution (1.5ml). The vial was heated for 15 minutes at 170°C, using a Personal Chemistry Smith™ synthesizer and cooled to room temperature. The mixture was partitioned between hydrochloric acid (2N, 15ml) and ethyl acetate (20 ml) and the phases were separated. After one more extraction the organic phases were combined, dried over sodium sulphate, filtered and evaporated. Purification by mass-triggered HPLC gave the title compound (30mg).
MS: 421 (ES (M-1-CO₂)).

### Example 69

### 3-(4-(Trifluoromethyl)phenyl)-4-(2-azido-4-(trifluoromethyl)phenyl)phenylacetic acid Step1:

### ethyl 3- (4- (trifluoromethyl)phenyl)-4-(2-amino-4-(trifluoromethyl)phenyl)phenylacetate

A mixture of 2-bromo-5-(trifluoromethyl)aniline (240mg, 1mmol), bis(pinacolato)diboron (305mg, 1.2mmol), potassium acetate (295mg, 3mmol) and [1,1'-diphenylphosphino)ferrocene] dichloropalladium(II), complex with dichloromethane (50mg) in dioxan was heated at 160°C for 15 minutes using a Personal Chemistry Smith™ synthesizer and cooled to room temperature. The reaction mixture was added to a vial containing the product from Example 68 Step 3 (1 mmol), [1,1'-diphenylphosphino)ferrocene] dichloropalladium(II), complex with dichloromethane (50mg) and saturated sodium carbonate solution (2ml). This was heated at 170°C for 15 minutes using a Personal Chemistry Smith™ synthesizer and cooled to room temperature. The mixture was partitioned between hydrochloric acid and ethyl acetate and the phases were separated. After extracting with ethyl acetate, the organic phases were combined, dried over sodium sulphate, filtered and evaporated. Purification by flash chromatography on silica using 5-10% ethyl acetate in isohexane as eluant yielded the title compound (112mg).

### Step2:

### ethyl 3-(4-(trifluoromethyl)phenyl)-4-(2-azido-4-(trifluoromethyl)phenyl)phenylacetate

The product from Step 1 (233mg,0.5mmol)in trifluoroacetic acid (5ml) was cooled to 0°C and treated with sodium nitrite (35mg, 0.5mmol). After 1 hour further sodium nitrite (35mg, 0.5mmol) was added. After1 hour sodium azide (65mg, 1mmol) was added. The reaction was allowed to warm to room temperature for 1 hour and stirred at room temperature for 1 hour. Water (20ml) added and the mixture was extracted using ethyl acetate (3x30ml). The combined organics were dried over sodium sulphate, filtered and evaporated. Purification by flash chromatography on silica using 10% ethyl acetate in isohexane as eluant gave the title compound (130mg).

### Step 3

### 3-(4-(trifluoromethyl)phenyl)-4-(2-azido-4- trifluoromethyl)phenyl)phenylacetic acid

The ester from Step 2 (50mg) in dioxan was treated with 40% potassium hydroxide solution (0.5ml) and was heated at 100°C for 18hours. The reaction mixture was cooled to room temperature, acidified using hydrochloric acid (2N) and extracted using ethyl acetate (50ml). The organic phase was dried over sodium sulphate, filtered and evaporated. Purification by mass-triggered HPLC gave the title compound (24mg). ¹H NMR (500 MHz, CDCl₃): d 3.78 (2H, s), 7.21-7.34 (6H, m), 7.38 (1H, s), 7.42 (1H, d, J= 7.2Hz), 7.48 (2H, d, J=8.0Hz)ppm.
MS: 392 (ES (M-1-CO₂-N₂)).

### Example 70

### 2-[4-(2-azido-4-(trifluoromethyl)phenyl)-3-(4-(trifluoromethyl)phenyl)phenyl]-4-methylpentanoic acid

### Step 1:

### methyl 2-[4-iodo-3-(4-(trifluoromethyl)phenyl)phenyl]-4-methylpentanoate

Ethyl 4-iodo-3-(4-(trifluoromethyl)phenyl)phenylacetate (Example 68 Step 3) was hydrolysed as in Example 69 Step 3 to give the corresponding acid. This was alkylated as described in Example 33, using 1-iodo-2-methylpropane in place of iodomethane and lithium diisopropylamide as base in place of butyl lithium to give 2-[4-iodo-3-(4-(trifluoromethyl)phenyl)phenyl]-4-methylpentanoic acid. This acid (1.59g,3.45mmol) in THF at 0°C was treated trimethylsilyldiazomethane (2M in hexanes,5ml, 10mmol). After 40 minutes, water (10ml) was added and nitrogen effluxed. The mixture was extracted using ethyl acetate (2x40ml). The combined organics were dried over sodium sulphate, filtered and evaporated. Purification by flash chromatography on silica using 0-2% ethyl acetate in isohexane as eluant gave the title compound (1.44g).

### Steps 2-4

The product of Step 1 was converted to the title compound by the procedure of Example 69. ¹H NMR (500 MHz, CDCl₃): d 0.97 (6H, d, J=6.6Hz), 1.55-1.65 (1H, m) 1.73-1.79 (1H, m), 2.04-2.10 (1H, m), 3.80 (1H, t, J=7.7Hz), 7.21 -7.31 (6H, m), 7.40 (1H, s), 7.45-7.49 (3H, m) ppm.
MS: 448 (ES (M-1-CO₂-N₂)).

### Example 71

### 4-(4-Methyl-1-(4-trifluoromethylphenyl)pent-1-ene-1-yl)-3-(4-(trifluoromethyl)phenyl)phenylacetic acid

### Step 1

### 3-Methyl-1-(tributylstannyl)-1-(4-trifluommethylphenyl)-1-butene

4-Iodobenzotrifluoride (11.587g 42.6mmol) was dissolved in pyrrolidine (130ml), then the solution was vacuum degassed and placed under nitrogen. Tetrakis triphenylphosphine palladium(0) (1.39g, 1.2mmol) was added followed by 4-methyl-I-pentyne (10ml, 85mmol). The reaction was stirred for 3.5 hrs at room temperature. Copper (I) iodide (436mg, 2.29mmol) was added, the reaction warmed up, then stirred for 1 day. Saturated ammonium chloride solution (300ml) was added, followed by extraction with ether (5x70ml). The combined organic extracts were washed with 1.7M hydrochloric acid (3x30ml) then water and dried over magnesium sulphate. The solvent was evaporated to leave an oil which was distilled at 75-78°C and 2mm Hg. Yield of 4-methyl 1-1-(4-trifluoromethylphenyl)- 1-pentyne 8g (83%).
The aforesaid alkyne (7.08 g, 31.3 mmol) was dissolved in THF (50 ml) and bis(triphenylphosphino)palladium(II)dichloride (1 g, 1.4 mmol) was added. The mixture was degassed three times and tributyltin hydride was added dropwise at room temperature. The black solution was stirred for an additional 4 hours and put onto water. The product was extracted with 3 portions (100 ml) of diethyl ether, the organic phases were combined, washed with brine and dried over sodium sulfate. After filtration and evaporation of the solvent, the remaining liquid was distilled under vacuum (0.05 mbar) to yield the title compound (13.5 g, 83 %) as a clear liquid, boiling point: 125 °C.
¹H NMR (500 MHz, CDCl3) δ 0.85 - 1.65 (34H, m), 1.89 (1H, t, J = 7.0 Hz), 5.81 (1H, t, J = 7.0 Hz), 7.00 (2H, d, J = 8.0 Hz), 7.50 (2H, d, J = 8.0 Hz) ppm.

### Step 2

Ethyl 4-iodo-3-(4-trifluoromethylphenyl)phenylacetate (Example 68 Step 3, 2.05g, 4.7mmol), 3-methyl-1-(tributylstannyl)-1-(4-trifluoromethylphenyl)-1-butene (Step 1, 3.67g, 7.1 mmol), tris(dibenzylidene)dipalladium(0) (0.15 g, 0.16 mmol), triphenylarsine (0.16 g, 0.52 mmol) and copper(I) iodide (0.09 g, 0.47 mmol) were dissolved in DMF. The solution was degassed three times and heated at 90°C for 15 hours. The black mixture was partitioned between water (10 ml) and ethyl acetate (50 ml) and the phases were separated. After two more extractions the organic phases were combined, dried over sodium sulphate, filtered and evaporated. Purification by flash chromatography on silica using 5-10% ethyl acetate in isohexane as eluant yielded ethyl 4-(4-methyl-1-(4-trifluoromethylphenyl)pent-1-ene-1-yl)-3-(4-(trifluoro-methyl)phenyl)phenylacetate (1.02g, 41 %) as an oil.
This ester (1.02g, 1.9 mmol) was dissolved in 5 ml ethanol and potassium hydroxide (1 g, 17.8 mmol) and water (0.5 ml) were added. The mixture was heated at 100°C for 12 hours. After cooling to room temperature, 1N HCl was added to pH of 4 and the aqueous phase was extracted with ethyl acetate (2 times 20mL). The organic phases were combined, dried over sodium sulphate, filtered and evaporated. Purification by flash chromatography on silica using 10 to 50% ethyl acetate in isohexane as eluant yielded 0.8 g (83%) of the target compound as a colourless oil.
¹H NMR (360 MHz, CDCl₃) δ 0.83 (6H, d, J = 6.4 Hz), 1.59 - 1.66 (1H, m), 2.02 (2H, d, J = 6.9 Hz), 3.69 (2H, s), 5.89 (1H, t, J = 7.4 Hz), 6.79 (2H, d, J = 7.7 Hz), 7.10 - 7.12 (3H, m), 7.24 (2H, d, J = 7.2 Hz), 7.37 - 7.40 (4H, m), 10.17 (1H, s, br.) ppm.
MS: (ES (M-1-CO₂)) 461.

### Example 72

### 4-[bis(3-methylbut-2-en-1-yl)amino]-3-[4-(trifluoromethyl)phenyl]phenylacetic acid

Ethyl 4-amino-3-(4-(trifluoromethyl)phenyl)phenylacetate hydrochloride salt (Example 68 Steps 2 and 3) (1.08g, 3mmol) in acetonitrile (50ml) was treated with potassium carbonate (1.66g, 12mmol). After 10 minutes, excess 1-bromo-3-methylbut-2-ene was added. The reaction mixture was heated at 60°C for 18 hours. The mixture was evaporated to give the crude ester (1.37g). The ester (0.86g) in dioxan (10ml) was treated with 40% potassium hydroxide solution (5ml) and was heated at 100°C for 2 hours. The reaction mixture was cooled to room temperature, neutralised using hydrochloric acid (2N) and extracted using ethyl acetate (2x30ml). The combined organics were dried over sodium sulphate, filtered and evaporated. Purification by mass-triggered HPLC gave the title compound (144mg). ¹H NMR (500 MHz, CDCl₃): d 1.48 (6H,s), 1.67 (6H, s), 3.69 (2H, s), 3.84 (4H, d, J=7.0Hz), 5.03 (2H, t, J = 7.0Hz), 7.17 (1H, d, J= 1.8Hz), 7.33 (1H, d, J=8.3Hz), 7.39-7.47 (3H, m), 7.69 (2H, d, J=8.1Hz) ppm.
MS: 432(ES (MH+).

## Claims

1. The use, for the manufacture of a medicament for treatment or prevention of a disease associated with the deposition of β-amyloid in the brain, of a compound of formula I: 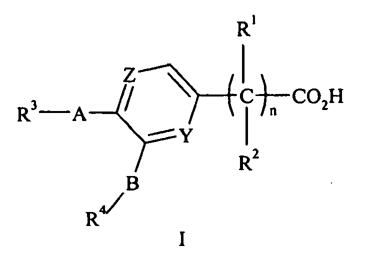 or a pharmaceutically acceptable salt thereof;
wherein n is 0, 1 or 2;
Y is N or CH;
Z is N or CR⁶;
A and B independently represent a bond or a divalent linking group comprising a chain of 1-3 atoms selected from C, O, N and S, with the proviso that not more than one of said atoms is O,N or S;
R¹ and R² independently represent H, F, OR⁵ or R⁵, or together complete a C₃₋₆cycloalkyl group which optionally bears a C₁₋₄alkyl substituent;
R³ and R⁴ independently represent acyclic hydrocarbon groups of 5-10 carbon atoms or mono-or bi-cyclic ring systems comprising 5 to 10 ring atoms selected from C, N, O and S, provided that not more than 3 ring atoms in any single ring are other than C, said ring system optionally bearing up to 3 substituents selected from halogen, N₃, CN, NO₂ R⁵, OR⁵, SR⁵, CO₂R⁵, OCOR⁵ and COR⁵;
R⁵ represents a hydrocarbon group of up to 7 carbon atoms which is optionally substituted with up to 3 halogen atoms; and
R⁶ represents H, or has the same definition as R³.

2. Use according to claim 1 wherein the disease associated with deposition of Aβ in the brain is Alzheimer's disease (AD), cerebral amyloid angiopathy, multi-infarct dementia, dementia pugilistica or Down syndrome.

3. A compound of formula I as defined in claim 1 or a pharmaceutically acceptable salt thereof, with the proviso that when n is 1 or 2, Y and Z are CH, and A and B both represent a bond, and at least one of R¹ and R² is H, then R³ and R⁴ do not both represent unsubstituted phenyl.

4. A compound according to claim 3 of Formula II: 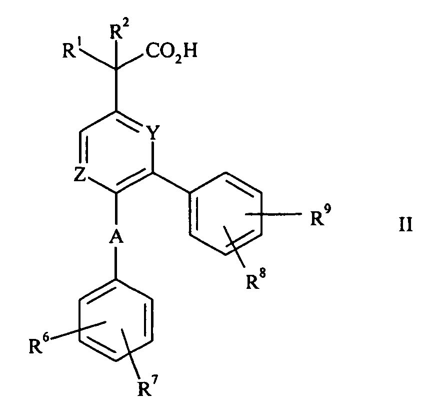 or a pharmaceutically acceptable salt thereof;
wherein R⁶, R⁷, R⁸ and R⁹ are independently selected from H, halogen, R⁵ and OR⁵ provided at least one of R⁶-R⁹ is other than H;
and A, Y, Z, R¹, R² and R⁵ are as defined in claim 3.

5. A compound according to claim 4 wherein R⁶ represents CF₃ which is in the 4-position and R⁷ represents H, halogen, N₃, C₁₋₄alkyl or CF₃.

6. A compound according to claim 4 or claim 5 wherein R⁸ represents CF₃ which is in the 4-position and R⁹ represents H, halogen or CF₃.

7. A compound according to any of claims 3-6 in which Y and Z are both CH.

8. A compound according to any of claims 3-7 in which A is a bond.

9. A compound according to any of claims 4-8 in which one of R¹ and R² is H and the other is H, R⁵ or OR⁵, or R¹ and R² together complete a cycloalkyl group.

10. A compound according to claim 9 in which R¹ is H and R² is C₁₋₆alkyl, haloC₁₋₆alkyl or C₂₋₆alkenyl.

11. A compound according to claim 10 which is selected from:
2-(3,4-Bis-(4-(trifluoromethyl)phenyl)phenyl)hexanoic acid;
2-(3,4-Bis-(4-(trifluoromethyl)phenyl)phenyl)butanoic acid;
2-(3,4-Bis-(4-(trifluoromethyl)phenyl)phenyl)pentanoic acid;
2-(3,4-Bis-(4-(trifluoromethyl)phenyl)phenyl)-3-methylbutanoic acid;.
2-(3,4-Bis-(4-(trifluoromethyl)phenyl)phenyl)-4-methylpentanoic acid;
2-(3,4-Bis-(4-(trifluoromethyl)phenyl)phenyl)-3-cyclopropylpropanoic acid;
2-(3,4-Bis-(4-(trifluoromethyl)phenyl)phenyl)-5,5,5 trifluoropentanoic acid;
2-(3,4-Bis-(4-(trifluoromethyl)phenyl)phenyl)-6-chlorohexanoic acid;
2-(3,4-Bis-(4-(trifluoromethyl)phenyl)phenyl)-4-methylpent-4-enoic acid;
and the pharmaceutically acceptable salts thereof.

12. A pharmaceutical composition comprising a compound according to any of claims 3-11 and a pharmaceutically acceptable carrier.

13. A compound according to any of claims 3-11 for use in retarding, preventing or arresting the accumulation of Aβ in the brain

## Patentansprüche

1. Verwendung einer Verbindung mit der Formel I für die Herstellung eines Medikaments zur Behandlung oder Verhütung einer Krankheit, die mit der Ablagerung von β-Amyloid im Gehirn verbunden ist: 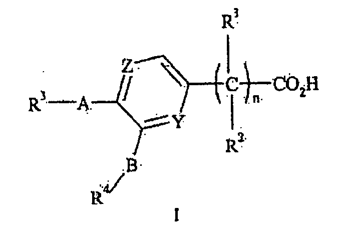 oder eines pharmazeutisch akzeptierbaren Salzes dieser Verbindung;
wobei
n gleich 0, 1 oder 2 ist;
Y für N oder CH steht;
Z für N oder CR⁶ steht;
A und B unabhängig voneinander eine Bindung oder eine zweiwertige Bindungsgruppe darstellen, die eine Kette von 1-3 Atomen aufweist, die unter C, O, N und S ausgewählt sind, unter der Bedingung, daß nicht mehr als eines der Atome O, N oder S ist;
R¹ und R² unabhängig voneinander H, F, OR⁵ oder R⁵ darstellen oder zusammen eine C₃₋₆-Alkylgruppe vervollständigen, die wahlweise einen C₁₋₄-Alkylsubstituenten trägt;
R³ und R⁴ unabhängig voneinander acyclische Kohlenwasserstoffgruppen von 5-10 Kohlenstoffatomen oder mono- oder bicyclische Ringsysteme mit 5 bis 10 Ringatomen darstellen, die unter C, N, O und S ausgewählt sind, vorausgesetzt, daß nicht mehr als 3 Ringatome in jedem einzelnen Ring andere als C-Atome sind, wobei das Ringsystem wahlweise bis zu 3 Substituenten trägt, die unter Halogen, N₃, CN, NO₂, R⁵, OR⁵, SR⁵, CO₂R⁵, OCOR⁵ und COR⁵ ausgewählt sind;
R⁵ eine Kohlenwasserstoffgruppe von bis zu 7 Kohlenstoffatomen darstellt, die wahlweise mit bis zu 3 Halogenatomen substituiert ist; und
R⁶ für H steht oder die gleiche Definition wie R³ hat.

2. Verwendung nach Anspruch 1, wobei die mit der Ablagerung von Aβ im Gehirn verbundene Krankheit die Alzheimersche Krankheit (AD), zerebrale Amyloidangiopathie, Multiinfarkt-Demenz, Dementia Pugilista oder das Down-Syndrom ist.

3. Verbindung gemäß Formel I, wie in Anspruch 1 definiert, oder ein pharmazeutisch akzeptierbares Salz davon, unter der Bedingung, daß, wenn n gleich 1 oder 2 ist, Y und Z für CH stehen und A und B beide eine Bindung darstellen und mindestens eine der Komponenten R¹ und R² für H steht, R³ und R⁴ nicht beide ein nichtsubstituiertes Phenyl darstellen.

4. Verbindung nach Anspruch 3 mit der Formel II: 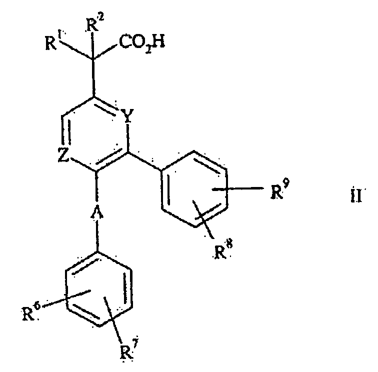 oder ein pharmazeutisch akzeptierbares Salz davon;
wobei R⁶, R⁷, R⁸ und R⁹ unabhängig voneinander unter H, einem Halogen, R⁵ und OR⁵ ausgewählt sind, vorausgesetzt, daß mindestens eine der Komponenten R⁶ - R⁹ nicht H ist;
und A, Y, Z, R¹, R² und R⁵ der Definition in Anspruch 3 entsprechen.

5. Verbindung nach Anspruch 4, wobei R⁶ für CF₃ steht, das sich in der 4-Stellung befindet, und R⁷ für H, ein Halogen, N₃, ein C₁₋₄-Alkyl oder CF₃ steht.

6. Verbindung nach Anspruch 4 oder Anspruch 5, wobei R⁸ für CF₃ steht, das sich in der 4-Stellung befinden, und R⁹ für H, ein Halogen oder CF₃ steht.

7. Verbindung nach einem der Ansprüche 3 - 6, in der Y und Z beide CH sind.

8. Verbindung nach einem der Ansprüche 3 - 7, in der A eine Bindung ist.

9. Verbindung nach einem der Ansprüche 4 - 8, in der eine der Komponenten R¹ und R² für H steht und die andere für H, R⁵ oder OR⁵ steht oder R¹ und R² zusammen eine Cycloalkylgruppe vervollständigen.

10. Verbindung nach Anspruch 9, in der R¹ für H steht und R² für ein C₁₋₆-Alkyl, ein Halogen-C₁₋₆-alkyl oder C₂₋₆-Alkenyl steht.

11. Verbindung nach Anspruch 10, die unter den folgenden Verbindungen ausgewählt ist:
2-(3,4-Bis-(4-(trifluormethyl)phenyl)phenyl)hexansäure;
2-(3,4-Bis-(4-(trifluormethyl)phenyl)phenyl)butansäure;
2-(3,4-Bis-(4-(trifluormethyl)phenyl)phenyl)pentansäure;
2-(3,4-Bis-(4-(trifluormethyl)phenyl)phenyl)-3-methylbutansäure;
2-(3,4-Bis-(4-(trifluormethyl)phenyl)phenyl)-4-methylpentansäure;
2-(3,4-Bis-(4-(trifluormethyl)phenyl)phenyl)-3-cyclopropylpropansäure;
2-(3,4-Bis-(4-(trifluormethyl)phenyl)phenyl)-5,5,5-trifluorpentansäure;
2-(3,4-Bis-(4-(trifluormethyl)phenyl)phenyl)-6-chlorhexansäure;
2-(3,4-Bis-(4-(trifluormethyl)phenyl)phenyl)-4-methylpent-4-ensäure;
und den pharmazeutisch akzeptierbaren Salzen davon,

12. Pharmazeutische Zusammensetzung, die eine Verbindung nach einem der Ansprüche 3 - 11 und einen pharmazeutisch akzeptierbaren Träger aufweist.

13. Verbindung nach einem der Ansprüche 3-11 zur Verwendung bei der Verzögerung, Verhütung oder Hemmung der Akkumulation von Aβ im Gehirn.

## Revendications

1. Utilisation, pour la fabrication d'un médicament pour le traitement ou la prévention d'une maladie associée au dépôt de β-amyloïde dans le cerveau, d'un composé de formule I: 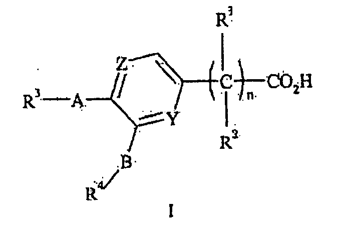 ou d'un sel pharmaceutiquement acceptable de celui-ci;
dans laquelle n est 0, 1 ou 2;
Y est N ou CH;
Z est N ou CR⁶;
A et B représentent indépendamment une liaison ou un groupe de liaison divalent comprenant une chaîne de 1-3 atomes choisis parmi C, O, N et S, étant entendu que pas plus d'un desdits atomes est O, N ou S;
R¹ et R² représentent indépendamment H, F, OR⁵ ou R⁵, ou achèvent ensemble un groupe cycloalkyle en C₃₋₆ qui porte facultativement un substituant alkyle en C₁₋₄;
R³ et R⁴ représentent indépendamment des groupes hydrocarbonés acycliques de 5-10 atomes de carbone ou des systèmes à noyau mono- ou bi-cyclique comprenant 5 à 10 atomes cycliques choisis parmi C, N, O et S, étant entendu que pas plus de 3 atomes cycliques dans tout noyau unique sont autres que C, ledit système cyclique portant facultativement jusqu'à 3 substituants choisis parmi halogène, N₃, CN, NO₂, R⁵, OR⁵, SR⁵, CO₂R⁵, OCOR⁵ et COR⁵;
R⁵ représente un groupe hydrocarboné à jusqu'à 7 atomes de carbone qui est facultativement substitué par jusqu'à 3 atomes d'halogène; et
R⁶ représente H, ou a la même définition que R³.

2. Utilisation selon la revendication 1, dans laquelle la maladie associée au dépôt de Aβ dans le cerveau est une maladie d'Alzheimer (AD), une angiopathie amyloïde cérébrale, une démence vasculaire, une démence pugilistique ou un syndrome de Down.

3. Composé de formule 1 telle que définie dans la revendication 1 ou sel pharmaceutiquement acceptable de celui-ci, étant entendu que lorsque n est 1 ou 2, Y et Z sont CH, et A et B représentent tous les deux une liaison, et au moins l'un parmi R¹ et R² est H, alors R³ et R⁴ ne représentent pas tous les deux un phényle non substitué.

4. Composé selon la revendication 3, de formule II: 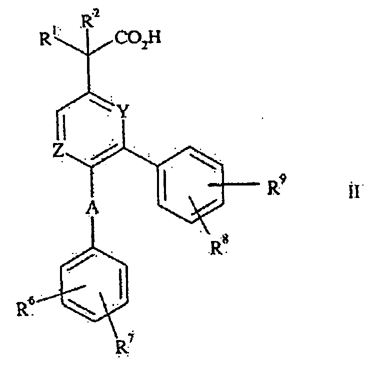 ou un sel pharmaceutiquement acceptable de celui-ci;
dans laquelle R⁶, R⁷, R⁸ et R⁹ sont indépendamment choisis parmi H, halogène, R⁵ et OR⁵, étant entendu qu'au moins l'un parmi R⁶ à R⁹ est autre que H;
et A, Y, Z, R¹, R² et R⁵ sont tels que définis dans la revendication 3.

5. Composé selon la revendication 4, dans lequel R⁶ représente CF₃ qui est en position 4 et R⁷ représente H, halogène, N₃, alkyle en C₁₋₄ ou CF₃.

6. Composé selon la revendication 4 ou la revendication 5, dans lequel R⁸ représente CF₃ qui est en position 4 et R⁹ représente H, halogène ou CF₃.

7. Composé selon l'une quelconque des revendications 3-6, dans lequel Y et Z sont tous les deux CH.

8. Composé selon l'une quelconque des revendications 3-7, dans lequel A est une liaison.

9. Composé selon l'une quelconque des revendications 4-8, dans lequel l'un parmi R¹ et R² est H et l'autre est H, R⁵ ou OR⁵, ou R¹ et R² achèvent ensemble un groupe cycloalkyle.

10. Composé selon la revendication 9, dans lequel R¹ est H et R² est alkyle en C₁₋₆, halogéno-alkyle en C₁₋₆ ou alcényle en C₂₋₆.

11. Composé selon la revendication 10, qui est choisi parmi:
l'acide 2-(3,4-bis-(4-(trifluorométhyl)phényl)phényl)hexanoïque;
l'acide 2-(3,4-bis-(4-(trifluorométhyl)phényl)phényl)butanoïque;
l'acide 2-(3,4-bis-(4-(trifluorométhyl)phényl)phényl)pentanoïque;
l'acide 2-(3,4-bis-(4-(trifluorométhyl)phényl)phényl)-3-méthylbutanoïque;
'acide 2-(3,4-bis-(4-(trifluorométhyl)phényl)phényl)-4-méthylpentanoïque;
l'acide 2-(3,4-bis-(4-(trifluorométhyl)phényl)phényl)-3-cyclopropylpropanoïque;
l'acide 2-(3,4-bis-(4-(trifluorométhyl)phényl)phényl)-5,5,5-trifluoropentanoïque;
l'acide 2-(3,4-bis-(4-(trifluorométhyl)phényl)phényl)-6-chlorohexanoïque;
l'acide 2-(3,4-bis-(4-(trifluorométhyl)phényl)phényl)-4-méthylpent-4-énoïque;
et les sels pharmaceutiquement acceptables de celui-ci.

12. Composition pharmaceutique comprenant un composé selon l'une quelconque des revendications 3-11 et un véhicule pharmaceutiquement acceptable.

13. Composé selon l'une quelconque des revendications 3-11 pour l'utilisation dans le retardement, la prévention ou l'arrêt de l'accumulation de Aβ dans le cerveau.
